# EUROPEAN PATENT SPECIFICATION

(11) **EP 3 820 841 B1**
(45) Date of publication and mention of the grant of the patent: **29.06.2022**
(21) Application number: 20757594.5
(22) Date of filing: 20.08.2020
(51) Int. Cl.: C07C 315/02, C07C 317/14

(54) **PROCESS FOR PRODUCING 4,4'-DICHLORODIPHENYL SULFONE**
VERFAHREN ZUR HERSTELLUNG VON 4,4-DICHLORDIPHENYL-SULFON
PROCÉDÉ DE PRODUCTION DE 4,4'-DICHLORODIPHÉNYL SULFONE

(30) Priority: 27.08.2019 EP 19193695
(43) Date of publication of application: 19.05.2021
(73) Proprietor: BASF SE, 67056 Ludwigshafen (DE)
(72) Inventor: GAO, Jun, 67056 Ludwigshafen (DE); THRUN, Frauke, 67056 Ludwigshafen (DE); THIEL, Indre, 67056 Ludwigshafen (DE); HAMANN, Jessica Nadine, 67056 Ludwigshafen (DE); SCHUETZ, Christian, 67056 Ludwigshafen (DE); BLEI, Stefan, 67056 Ludwigshafen (DE)
(74) Representative: BASF IP Association
(86) International application number: PCT/EP2020/073359
(87) International publication number: WO 2021/037675

(56) References cited:
- CN-A- 104 557 626
- CN-A- 108 047 101

## Description

The invention relates to a process for producing 4,4'-dichlorodiphenyl sulfone which also is called 1,1'-sulfonylbis(4-chlorobenzene) or bis(4-chlorophenyl) sulfone.

4,4'-dichlorodiphenyl sulfone (in the following DCDPS) is used for example as a monomer for preparing polymers like polyarylene(ether)sulfones such as polyether sulfone or polysulfone or as an intermediate of pharmaceuticals, dyes and pesticides.

DCDPS for example is produced by oxidation of 4,4'-dichlorodiphenyl sulfoxide (in the following DCDPSO) which can be obtained by a Friedel-Crafts reaction of thionyl chloride and chlorobenzene as starting materials in the presence of a catalyst, for example aluminum chloride.

Two stage processes in which in a first stage DCDPSO is obtained which in a second stage is oxidized to DCDPS in the presence of hydrogen peroxide and acetic acid are for instance disclosed in CN-A 108047101, CN-A 102351758, CN-B 104402780, CN-A 104557626 and SU-A 765262.

Further processes for obtaining DCDPS by reacting chlorobenzene and thionyl chloride in a Friedel-Crafts reaction in a first stage to obtain 4,4'-dichlorodiphenyl sulfoxide and to oxidize the 4,4'-dichlorodiphenyl sulfoxide in a second stage using hydrogen peroxide as oxidizing agent and dichloromethane or dichloropropane as solvent are disclosed in CN-A 102351756 and CN-A 102351757.

A process for producing an organic sulfone by oxidation of the respective sulfoxide in the presence of at least one peroxide is disclosed in WO-A 2018/007481. The reaction thereby is carried out in a carboxylic acid as solvent, the carboxylic acid being liquid at 40°C and having a miscibility gap with water at 40°C and atmospheric pressure.

General processes for the production of sulfur containing diaryl compounds are disclosed for example in Sun, X. et al, Journal of Chemical Research 2013, pages 736 to 744, Sun, X. et al, Phosphorus, Sulfur, and Silicon, 2010, Vol. 185, pages 2535-2542 and Sun, X. et al., 2017, Vol. 192, No. 3, pages 376 to 380. In these papers different reaction conditions and catalysts are compared.

It is an object of the present invention to provide a reliable and energy-efficient process for producing DCDPS with a reduced amount of impurities.

This object is achieved by a process for producing 4,4'-dichlorodiphenyl sulfone, comprising:
(I) reacting thionyl chloride, chlorobenzene and aluminum chloride in a molar ratio of thionyl chloride : chlorobenzene : aluminum chloride of 1 : (6 to 9) : (1 to 1.5) at a temperature in the range from 0 to below 20°C, forming an intermediate reaction product and hydrogen chloride;
(II) mixing aqueous hydrochloric acid and the intermediate reaction product at a temperature in the range from 70 to 110°C to obtain an organic phase comprising 4,4'-dichlorodiphenyl sulfoxide and an aqueous phase;
(III) cooling the organic phase comprising the 4,4'-dichlorodiphenyl sulfoxide to a temperature below the saturation point of 4,4'-dichlorodiphenyl sulfoxide to obtain a suspension comprising crystallized 4,4'-dichlorodiphenyl sulfoxide;
(IV) solid-liquid-separation of the suspension to obtain a residual moisture comprising solid 4,4'-dichlorodiphenyl sulfoxide comprising crystallized 4,4'-dichlorodiphenyl sulfoxide and solvent, and a first mother liquor;
(V) washing the residual moisture comprising solid 4,4'-dichlorodiphenyl sulfoxide with a carboxylic acid to obtain carboxylic acid-wet 4,4'-dichlorodiphenyl sulfoxide;
(VI) reacting the carboxylic acid-wet 4,4'-dichlorodiphenyl sulfoxide and an oxidizing agent in a carboxylic acid as solvent to obtain a reaction mixture comprising 4,4'-dichlorodiphenyl sulfone and carboxylic acid;
(VII) separating the reaction mixture comprising 4,4'-dichlorodiphenyl sulfone and carboxylic acid into a residual moisture comprising 4,4'-dichlorodiphenyl sulfone as crude product and a liquid phase comprising carboxylic acid,
(VIII) optionally working up the residual moisture comprising 4,4'-dichlorodiphenyl sulfone.

It is understood that each of the procedures (I) to (VII) comprised in this process may itself comprise additional process measures.

By this process 4,4'-dichlorodiphenyl sulfoxide is initially produced which contains less than 0.5 wt% isomers based on the total amount of all isomers of dichlorodiphenyl sulfoxide. This has the additional advantage, that 4,4'-dichlorodiphenyl sulfone is achieved which contains only a low amount of impurities.

It is a further advantage of this process that the residual moisture comprising solid DCDPSO is essentially free of aluminum chloride used as catalyst. "Essentially free" in this context means that, if at all detectable, there are only traces of aluminum chloride in the product obtained from the process, preferably, the amount of aluminum chloride is from 0 to 100 ppm, particularly less than 50 ppm. Thus, also the finally produced DCDPS is essentially free of aluminum chloride. The person skilled in the art appreciates that any indications of time periods given below may depend on parameters such as the amount of material handled.

"Inert gas" in context of the present application means non-oxidizing gases and is preferably nitrogen, carbon dioxide, noble gases like argon or any mixture of these gases. Particularly preferably, the inert gas is nitrogen.

"Saturation point" denotes the temperature of a DCDPSO or DCDPS comprising solution at which DCDPSO or DCDPS starts to crystallize. This temperature depends on the concentration of the DCDPSO or the DCDPS in the solution. The lower the concentration of DCDPSO or DCDPS in the solution, the lower is the temperature at which crystallization starts.

Each process step described below can be carried out in only one apparatus or in more than one apparatus depending on the apparatus size and the amounts of compounds to be added. If more than one apparatus is used for a process step, the apparatus can be operated simultaneously or - particularly in a batchwise operated process - at different time. Certain procedures, such as washing or crystallization can be carried out in one or more cycles. Generally, it is possible to carry out the process entirely or in part continuously or batchwise. To obtain DCDPSO, in the reaction (I) thionyl chloride, chlorobenzene and aluminum chloride are fed into a reactor in a molar ratio of thionyl chloride : chlorobenzene : aluminum chloride of 1 : (6 to 9) : (1 to 1.5), preferably in a molar ratio of thionyl chloride : chlorobenzene : aluminum chloride of 1 : (7 to 9) : (1 to 1.2), particularly in a molar ratio of thionyl chloride : chlorobenzene : aluminum chloride of 1 : (7 to 8) : (1 to 1.1).

The reactor can be any reactor which allows mixing and reacting of the components fed into the reactor. A suitable reactor is for example a stirred tank reactor or jet loop reactor. Preferably, the reaction is operated batchwise.

The thionyl chloride, chlorobenzene and aluminum chloride can be added simultaneously or successively. For reasons of ease of conduct of the reaction - in particular in case of batch reaction - preferably, aluminum chloride and chlorobenzene are fed firstly into the reactor and then the thionyl chloride is added to the aluminum chloride and chlorobenzene. In this case the aluminum chloride and chlorobenzene can be added simultaneously or one after the other. However, in each case it is preferred to mix the aluminum chloride and chlorobenzene before adding the thionyl chloride. Particularly preferably aluminum chloride and chlorobenzene are first fed into the reactor and the thionyl chloride is added to the aluminum chloride and chlorobenzene. During the reaction hydrogen chloride (HCI) - typically in gaseous form - is formed which is at least partially withdrawn from the reactor. The volumetric flow for adding the thionyl chloride typically depends on heat dissipation and flow rate of the gas withdrawn from the reactor.

The chlorobenzene which is added in excess into the reactor and, therefore, only partially converted during the chemical reaction, also serves as a solvent for the reaction products. In steps (I) to (IV) in which a solvent is used, the solvent preferably is chlorobenzene. Due to the reaction conditions in the context of the present invention the person skilled in the art appreciates that the term "chlorobenzene" means monochlorobenzene which may contain traces of impurities.

The thionyl chloride and the chlorobenzene react in the presence of the aluminum chloride whereby an intermediate reaction product and hydrogen chloride form. The intermediate reaction product comprises 4,4'-dichlorodiphenyl sulfoxide-AlCl₃ adduct. The aluminum chloride generally can act as catalyst. The chemical reaction can be schematically represented by the following chemical reaction equation (1):

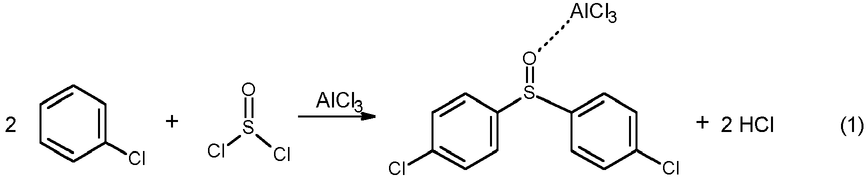

The reaction (I) is carried out at a temperature from 0 to below 20°C, preferably at a temperature from 3 to 15°C, particularly from 5 to 12°C.

The reaction period generally depends on the amounts of reactants used and increases with increasing amounts of reactants. After addition of the thionyl chloride to the mixture of aluminum chloride and chlorobenzene is completed, the reaction preferably is continued for 10 to 120 min, more preferred from 20 to 50 min after the total amount of thionyl chloride is fed into the reactor.

The hydrogen chloride (HCI) produced in the reaction typically is in gaseous form and at least partly removed from the reactor. While it can be put to other use in gaseous form, preferably, the hydrogen chloride removed from the reaction is mixed with water to produce aqueous hydrochloric acid.

After the reaction the intermediate reaction product is mixed with aqueous hydrochloric acid. For reasons of energy as well as production efficiency as well as sustainability, particularly preferably, the aqueous hydrochloric acid is produced from the hydrogen chloride removed from the reaction (I). By mixing the intermediate reaction product with the aqueous hydrochloric acid hydrolysis of the intermediate reaction product can take place. An organic phase comprising DCDPSO is obtained. The organic phase comprising DCDPSO can also comprise aluminum chloride which is typically in hydrated form, usually as AlCl₃·6H₂O. The hydrolysis can be schematically represented by reaction equation (2):

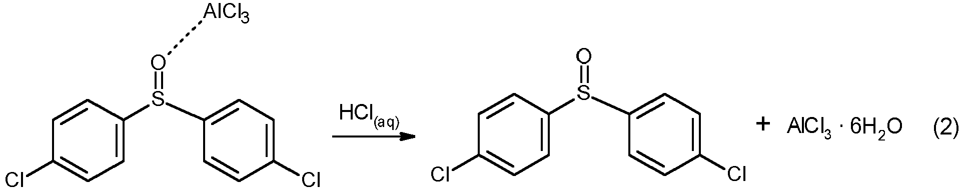

The temperature at which the hydrolysis is carried out is from 70 to 110°C, preferably from 80 to 100°C, particularly from 80 to 90°C. The reaction period of the hydrolysis after all components for the hydrolysis are added preferably is from 30 to 120 min, more preferred from 30 to 60 min, particularly from 30 to 45 min. This reaction period is generally sufficient for hydrolysis of the intermediate reaction product to obtain the DCDPSO. After finishing the hydrolysis, the mixture separates into an aqueous phase comprising the AlCl₃ and an organic phase comprising DCDPSO solved in the excess chlorobenzene.

The aqueous hydrochloric acid may have any concentration. However, a concentration of the hydrochloric acid above 3 wt% improves the solubility of the aluminum chloride. Preferably, the aqueous hydrochloric acid used in the hydrolysis has a concentration from 3 to 12 wt%, more preferably from 6 to 12 wt%, particularly from 10 to 12 wt%. All concentrations of hydrochloric acid in wt% above and in the following are based on the total amount of hydrogen chloride and water in the aqueous hydrochloric acid. An advantage of a higher concentration, particularly of a concentration from 10 to 12 wt% is that the density of the aqueous phase increases and the aqueous phase thus forms the lower phase whereas the upper phase is the organic phase comprising the DCDPSO, in the following also termed as "DCDPSO comprising organic phase". This allows an easier draining of the aqueous phase to obtain the DCDPSO comprising organic phase. Further, the higher concentration allows a smaller amount of water for removing the aluminum chloride. A higher concentration of the aqueous hydrochloric acid further results in a quicker phase separation.

The amount of aqueous hydrochloric acid used in (II) preferably is such that no aluminum chloride precipitates and that further two liquid phases are formed, the lower phase being the aqueous phase and the DCDPSO comprising organic phase being the upper phase. To achieve this, the amount of aqueous hydrochloric acid added to the reaction mixture preferably is such that after the hydrolysis the weight ratio of aqueous phase to DCDPSO comprising organic phase is from 0.6 to 1.5 kg/kg, more preferably from 0.7 to 1.0 kg/kg, particularly from 0.8 to 1.0 kg/kg. A smaller amount of aqueous hydrochloric acid may result in precipitation of aluminum chloride. Particularly at higher concentrations of the aqueous hydrochloric acid a larger amount is necessary to avoid precipitation. Therefore, the concentration of the aqueous hydrochloric acid preferably is kept below 12 wt%.

If the reaction and the hydrolysis are carried out in the same reactor, the aqueous hydrochloric acid is fed into the reactor after the reaction is completed and after the intermediate reaction product is heated to the temperature of the hydrolysis. The flow rate of the aqueous hydrochloric acid preferably is set such that the temperature of the hydrolysis can be held in the specified range for the hydrolysis by tempering the reactor. If the reaction and the hydrolysis are carried out in different reactors, it is preferred to firstly feed the aqueous hydrochloric acid into the second reactor and to add the intermediate reaction product to the aqueous hydrochloric acid. In this case the flow rate of adding the intermediate reaction product into the second reactor is set such that the temperature in the second reactor is held within the specified temperature limits for the hydrolysis by tempering the second reactor.

To remove the aqueous hydrochloric acid and remainders of the aluminum chloride from the DCDPSO comprising organic phase, the DCDPSO comprising organic phase obtained in (II) preferably is separated off and washed with an extraction liquid before cooling in (III). The phase separation following the hydrolysis can be carried out in the reactor in which the hydrolysis took place or in a separate vessel for phase separation. Using aqueous hydrochloric acid having a higher concentration for removing aluminum chloride, e.g. from 10 to 12 wt% has the advantage that the washing of the DCDPSO comprising organic phase can be carried out in the same apparatus as the hydrolysis.

The extraction liquid used for washing the DCDPSO comprising organic phase preferably is water. Particularly preferably, the water which is used for washing the DCDPSO comprising organic phase is separated off after washing and mixed with the hydrogen chloride obtained in (I) to obtain the aqueous hydrochloric acid.

The washing preferably is carried out at a temperature from 70 to 110°C, more preferred from 80 to 100°C, particularly from 80 to 90°C. Particularly preferably the washing is carried out at the same or essentially the same temperature as the hydrolysis.

Generally, the amount of extraction liquid which preferably is water is sufficient to remove all or essentially all of the aluminum chloride from the DCDPSO comprising organic phase. The amount of water used for washing preferably is chosen in such a way that a weight ratio of aqueous phase to DCDPSO comprising organic phase from 0.3 to 1.2 kg/kg, more preferably from 0.4 to 0.9 kg/kg, particularly from 0.5 to 0.8 kg/kg is obtained. In terms of sustainability and avoidance of large waste water streams it is preferred to use as little water for the washing step as possible. It is particularly preferred to use such an amount of water, that the entire aqueous phase from the washing step can be used to generate the aqueous hydrochloric acid in the concentration needed for hydrolysis. For this purpose, the water which is used for washing is separated off and mixed with the hydrogen chloride obtained in the reaction to obtain the aqueous hydrochloric acid.

After a predetermined washing period, mixing is stopped to allow the mixture to separate into an aqueous phase and an organic phase. The organic phase comprises the DCDPSO solved in the excess chlorobenzene as solvent.

For separating the DCDPSO from the DCDPSO comprising organic phase, the DCDPSO comprising organic phase is cooled to a temperature below the saturation point of DCDPSO in (III) to obtain a suspension comprising crystallized DCDPSO (in the following also termed as "DCDPSO comprising suspension").

The cooling (III) for crystallizing DCDPSO can be carried out in any crystallization apparatus or any other apparatus which allows cooling of the DCDPSO comprising organic phase, for example an apparatus with surfaces that can be cooled such as a vessel or a tank with cooling jacket, cooling coils or cooled baffles like so called "power baffles".

To avoid precipitation and fouling on cooled surfaces, it is preferred that cooling is carried out in a gastight closed vessel by a procedure which comprises:
(III.a) reducing the pressure in the gastight closed vessel;
(III.b) evaporating solvent;
(III.c) condensing the evaporated solvent by cooling;
(III.d) returning the condensed solvent into the gastight closed vessel.

It is preferred to use a gastight closed vessel without cooled surfaces whereby formation of a solid surface layer of crystallized DCDPSO can be reduced or avoided.

To crystallize DCDPSO, it is necessary to provide crystal nuclei. To provide the crystal nuclei it is possible to use dried crystals which are added to the DCDPSO comprising organic phase or to add a suspension comprising particulate DCDPSO as crystal nuclei. If dried crystals are used but the crystals are too big, it is possible to grind the crystals into smaller particles which can be used as crystal nuclei. Further, it is also possible to provide the necessary crystal nuclei by applying ultrasound to the DCDPSO comprising organic phase. Preferably, the crystal nuclei are generated in situ in an initializing step. The initializing step preferably comprises following steps before setting the reduced pressure in step (III.a):
- reducing the pressure in the gastight closed vessel such that the boiling point of the DCDPSO comprising organic phase is from 80 to 95°C;
- evaporating solvent until an initial formation of solids takes place;
- increasing the pressure in the vessel and heating the DCDPSO comprising organic phase in the vessel to a temperature from 85 to 100°C.

By reducing the pressure in the vessel such that the boiling point of the DCDPSO comprising organic phase is from 80 to 95°C, preferably from 83 to 92°C the following evaporation of solvent leads to a saturated solution and the precipitation of DCDPSO. By the following pressure increase and heating the DCDPSO comprising organic phase in the gastight closed vessel to a temperature from 85 to 100°C the solidified DCDPSO starts to partially dissolve again. This has the effect that the number of crystal nuclei is reduced which allows producing a smaller amount of crystals with a bigger size.

For generating the crystal nuclei in the initializing step, it is possible to only evaporate solvent until an initial formation of solids take place. It is also possible to entirely condense the evaporated solvent by cooling and to return all the condensed solvent into the gastight closed vessel. The latter has the effect that the liquid in the gastight closed vessel is cooled and solid forms. A mixture of both approaches, where only a part of the evaporated and condensed solvent is returned into the gas tight vessel, is also viable.

The pressure in step (III.a) preferably is reduced until the temperature in the gastight closed vessel reaches a predefined value from 0 to 45°C, more preferably from 10 to 35°C, particularly from 20 to 30°C. At these predefined temperatures the pressure in the gastight closed vessel typically is from 20 to 350 mbar(abs), preferably from 20 to 200 mbar(abs), particularly from 20 to 100 mbar(abs). After the predefined temperature value is reached, pressure reduction is stopped and then the gastight closed vessel is vented until ambient pressure is reached. The temperature profile in the gastight closed vessel preferably is selected such that the DCDPSO comprising organic phase is subjected to a constant supersaturation.

To reduce the solubility of the DCDPSO and thus increase the yield of solidified DCDPSO it is necessary to shift the saturation point. This is possible by continuously reducing the amount of solvent at a constant temperature, for example by evaporating solvent, or by cooling the DCDPSO comprising organic phase at constant concentration. Since reduction of the amount of solvent results in a very viscous suspension when a certain critical concentration is reached, it is preferred to increase the yield of solidified DCDPSO partly by reducing the amount of solvent by evaporation followed by reducing the temperature. For reducing the solubility of DCDPSO in the DCDPSO comprising organic phase and to improve the crystallization, it is possible to additionally add at least one drowning-out agent, for example at least one protic solvent like water, an alcohol, and/or an acid, particularly a carboxylic acid, or at least one highly unpolar solvent like a linear and/or cyclic alkane. With respect to ease of workup water, methanol, ethanol, acetic acid and/or formic acid, particularly water and/or methanol are preferred drowning-out agents.

After reaching ambient pressure the DCDPSO comprising suspension which formed in the gastight closed vessel by the cooling is withdrawn and fed into the solid-liquid-separation (IV).

Crystallization preferably is continued until the solids content in the DCDPSO comprising suspension in the last step of the crystallization is from 5 to 50 wt%, more preferred from 5 to 40 wt%, particularly from 20 to 40 wt%, based on the mass of the DCDPSO comprising suspension.

Even though the cooling and crystallization can be carried out continuously or batchwise, it is preferred to carry out the cooling and crystallization batchwise. Batchwise cooling and crystallization allows a higher flexibility in terms of operating window and crystallization conditions and is more robust against variations in process conditions.

Independently of whether the cooling and crystallization is performed continuously or batchwise, the solid-liquid-separation (IV) can be carried out either continuously or batchwise, preferably continuously.

The solid-liquid-separation (IV) for example comprises a filtration, centrifugation or sedimentation. Preferably, the solid-liquid-separation is a filtration. In the solid-liquid-separation (IV) a first mother liquor is removed from the solid DCDPSO and residual moisture comprising DCDPSO (in the following also termed as "moist DCDPSO") is obtained. If the solid-liquid-separation is a filtration, the moist DCDPSO is called "DCDPSO filter cake".

The solid-liquid-separation preferably is performed at ambient temperature or temperatures below ambient temperature, preferably at ambient temperature.

To carry out the solid-liquid-separation (IV) any solid-liquid-separation apparatus known by the skilled person can be used.

As by cooling the majority of DCDPSO crystallizes but still a considerable amount of the DCDPSO remains dissolved in the solvent, the first mother liquor withdrawn from the solid-liquid-separation apparatus preferably is concentrated and at least a part of the concentrated first mother liquor is recycled into the cooling step (III). Concentration of the first mother liquor preferably is performed by distillation or evaporation, preferably by evaporation. By concentrating the first mother liquor and recycling the first mother liquor into the cooling step (III) it is possible to reduce product loss to a minimum.

The distillation or evaporation for concentrating the first mother liquor can be carried out either at ambient pressure or at reduced pressure, preferably at a pressure from 20 to 800 mbar(abs), preferably in a range from 50 to 500 mbar(abs), particularly in a range from 100 to 350 mbar(abs).

Evaporation or distillation preferably is continued until the concentration of DCDPSO in the first mother liquor is from 6 to 60 wt%, preferably from 10 to 50 wt%, particularly from 15 to 40 wt%, based on the total amount of concentrated first mother liquor. If the concentration of DCDPSO is below 6 wt% the loss of chlorobenzene is too high, and if the concentration of DCDPSO is above 60wt-% there is a risk of uncontrolled crystallization in the evaporators and/or distillation apparatus.

At least a part of the concentrated first mother liquor preferably is recycled into the cooling step (III). To avoid an excessive accumulation of high boiling byproducts and contaminants it is preferred to recycle a part of the concentrated first mother liquor into the cooling step (III) and to withdraw the rest of the concentrated first mother liquor from the process.

The recycled concentrated first mother liquor preferably is mixed with fresh DCDPSO comprising organic phase and fed into the cooling (III). The ratio of fresh DCDPSO comprising organic phase to concentrated first mother liquor preferably is from 60:1 to 6:1, preferably from 15:1 to 7:1, particularly from 10:1 to 7:1. The amount of concentrated first mother liquor recycled into the cooling (III) preferably is set such that the amount of isomers of DCDPSO, particularly the amount of 2,4'-dichlorodiphenyl sulfoxide, totally fed into the cooling (III) is in the range from 0 to 40 wt% and particularly in the range from 10 to 30 wt% based on the total amount of liquid fed into the cooling (III). An amount of isomers in this range does not have negative effects on the quality of the DCDPS produced from the DCDPSO.

By concentrating and recycling at least a part of the first mother liquor, the yield of DCDPSO usually can be increased considerably, such as up to about 10 %, an increase of at least about 8 or 9 % can be possible. This allows for carrying out the crystallization in only one step.

After solid-liquid-separation, the resulting moist DCDPSO preferably is washed with a washing liquid, particularly with solvent. By washing the moist DCDPSO with solvent, impurities which may attach to the surface of the crystallized DCDPSO can be removed. Using the solvent for washing the moist DCDPSO has the additional advantage that impurities adhering to the surface of the crystallized DCDPSO can be removed because the DCDPSO starts to solve at the surface and thus the impurities adhering to the surface loosen and can be removed.

To reduce the amount of solvent used in the process, preferably at least a part of the solvent is purified after being used for washing the moist DCDPSO and recycled. The purification of the solvent can be carried out by each process known by a person skilled in the art. Particularly suitable are distillation or evaporation processes to separate impurities from the solvent.

The thus purified solvent for example can be reused for washing the moist DCDPSO. Alternatively, it is also possible to recycle at least a part of the purified solvent into the reaction (I).

For washing, the DCDPSO filter cake is brought into contact with washing liquid, preferably with solvent.

Washing of the moist DCDPSO preferably is operated at ambient temperature. It is also possible to wash the moist DCDPSO at temperatures different to ambient temperature, for instance above ambient temperature. To avoid dissolving the DCDPSO in the solvent, it is preferred to keep the washing temperature at a temperature where the solubility of DCDPSO in the solvent is very low, preferably from 0 to 5 wt% based on the sum of DCDPSO and solvent.

To use the thus produced DCDPSO for producing DCDPS, it is necessary to remove the solvent, particularly the chlorobenzene, as chlorobenzene in the DCDPSO may result in toxic by-products during the formation of DCDPS. For removing the solvent, the moist DCDPSO is washed with a carboxylic acid in (V). Preferably washing with carboxylic acid is continued until the amount of solvent in the moist DCDPSO is below 1.5 wt% based on the total amount of the moist DCDPSO after washing.

An amount of solvent, particularly chlorobenzene, preferably below 1.5 wt%, particularly below 1 wt% allows to use the obtained composition comprising DCDPSO and carboxylic acid (in the following also termed as "carboxylic acid-wet DCDPSO") as such or DCDPSO isolated therefrom to produce 4,4'-dichlorodiphenyl sulfone without generating an explosive gas phase or liquid phase. Further, such a low amount of solvent reduces the formation of toxic by-product to an extent which has no detrimental effect on the further use of the 4,4'-dichlorodiphenyl sulfone produced by oxidation of the DCDPSO worked up according to the invention.

Washing of the moist DCDPSO can be carried out in any apparatus which allows washing of a residual moisture comprising compound. An apparatus which can be used for the washing for example is a stirred tank or filtration apparatus. If a filtration apparatus is used for washing, the amount of carboxylic acid used for washing the moist DCDPSO preferably is at least 0.15 times the total mass of the moist DCDPSO, more preferred at least 0.2 times the total mass of the moist DCDPSO, particularly at least 0.5 times the total mass of the moist DCDPSO. The maximum amount of carboxylic acid used for washing the moist DCDPSO preferably is 3 times the total mass of the moist DCDPSO, more preferred 2 times the total mass of the moist DCDPSO, particularly 1.5 times the total mass of the moist DCDPSO if a filtration apparatus is used for washing the moist DCDPSO. If a stirred tank is used for washing, the amount of carboxylic acid for washing preferably is between 0.5 and 3 times the total mass of the moist DCDPSO, more preferred 1 to 2 times the total mass of the moist DCDPSO, particularly 1 to 1.5 times the total mass of the moist DCDPSO.

If the washing (V) is carried out in a stirred tank, a solid-liquid-separation can take place after washing the moist DCDPSO. For solid-liquid-separation any operation known to a skilled person can be used. Suitable solid-liquid-separation operations for example are filtration or centrifugation. If the solid-liquid-separation is a filtration, any filtration apparatus can be used.

By means of the washing (V), solvent is replaced by carboxylic acid in the moist DCDPSO. The carboxylic acid-wet DCDPSO comprises DCDPSO, carboxylic acid and remainders of solvent in an amount of preferably less than 1.5 wt% based on the total amount of the carboxylic acid-wet DCDPSO, more preferred in an amount of less than 1.2 wt% based on the total amount of the carboxylic acid-wet DCDPSO, particularly in an amount of less than 1 wt% based on the total amount of the carboxylic acid-wet DCDPSO. The amount of carboxylic acid in the carboxylic acid-wet DCDPSO preferably is between 6 and 30 wt% based on the total mass of the carboxylic acid-wet DCDPSO, more preferred between 9 and 25 wt% based on the total mass of the carboxylic acid-wet DCDPSO, particularly between 9 and 15 wt% based on the total mass of the carboxylic acid-wet DCDPSO. The wt% ranges given above refer to the carboxylic acid-wet DCDPSO after the filtration has been carried out in a filtration apparatus or, if the washing is carried out in a stirred tank, after the solid-liquid separation following the washing.

During the washing (V) a liquid mixture comprising solvent and carboxylic acid is obtained and withdrawn from the washing apparatus. To reduce the amount of carboxylic acid and solvent to be disposed, it is preferred to separate the liquid mixture comprising solvent and carboxylic acid into a first stream comprising substantially solvent and a second stream comprising substantially carboxylic acid. This allows the first and second streams to be recycled or used in different processes which use either carboxylic acid or solvent. "Comprising substantially solvent" in this context means that the first stream comprises preferably at least 95 wt% solvent, more preferred at least 98 wt% solvent, particularly at least 99 wt% solvent, each based on the total amount of the first stream. The second stream preferably comprises at least 80 wt% carboxylic acid, more preferred at least 85 wt% carboxylic acid, particularly at least 88 wt% carboxylic acid, each based on the total amount of the second stream. Reason for the lower content of carboxylic acid in the second stream compared to the amount of solvent in the first stream is that the liquid mixture still contains a considerable amount of DCDPSO. This can for instance be an amount of about 10 wt% based on the total amount of the first stream. As the DCDPSO is a high boiler compared to the solvent, the DCDPSO also collects in the second stream.

It is particularly preferred to recycle the second stream comprising substantially carboxylic acid into the washing (V) of the moist DCDPSO. The first stream comprising substantially solvent preferably is recycled into the reaction (I).

Separation of the liquid mixture into the first and second streams can be obtained for example by distillation or evaporation. Evaporation or distillation generally is carried out at a pressure below ambient pressure, preferably at a pressure from 20 to 700 mbar(abs), more preferred from 50 to 500 mbar(abs), particularly in a range from 70 to 200 mbar(abs). Evaporation or distillation typically is carried out at a temperature above the boiling point of the solvent in the liquid mixture, preferably at a temperature from 130 to 200°C more preferred from 140 to 180°C, particularly in a range from 150 to 170°C in the bottom of the distillation column.

The carboxylic acid used for washing the moist DCDPSO can be only one carboxylic acid or a mixture of at least two different carboxylic acids. Preferably the carboxylic acid is at least one aliphatic carboxylic acid. The at least one aliphatic carboxylic acid may be at least one linear or at least one branched aliphatic carboxylic acid or it may be a mixture of one or more linear and one or more branched aliphatic carboxylic acids. Preferably the aliphatic carboxylic acid is an aliphatic C₆ to C₁₀ carboxylic acid, particularly a C₆ to Cg carboxylic acid, whereby it is particularly preferred that the at least one carboxylic acid is an aliphatic monocarboxylic acid. Thus, the at least one carboxylic acid may be hexanoic acid, heptanoic acid, octanoic acid nonanoic acid or decanoic acid or a mixture of one or more of said acids. For instance the at least one carboxylic acid may be n-hexanoic acid, 2-methyl-pentanoic acid, 3-methyl-pentanoic acid, 4-methyl-pentanoic acid, n-heptanoic acid, 2-methyl-hexanoic acid, 3-methyl-hexanoic acid, 4-methyl-hexanoic acid, 5-methyl-hexanoic acid, 2-ethyl-pentanoic acid, 3-ethyl-pentanoic acid, n-octanoic acid, 2-methyl-heptanoic acid, 3-methyl-heptanoic acid, 4-methyl-heptanoic acid, 5-methyl-heptanoic acid, 6-methyl-heptanoic acid, 2-ethyl-hexanoic acid, 4-ethyl-hexanoic acid, 2-propyl pentanoic acid, 2,5-dimethylhexanoic acid, 5,5-dimethyl-hexanoic acid, n-nonanoic acid, 2-ethyl-hepatnoic acid, n-decanoic acid, 2-ethyl-octanoic acid, 3-ethyl-ocantoic acid, 4-ethyl-octanoic acid. The carboxylic acid may also be a mixture of different structural isomers of one of said acids. For instance, the at least one carboxylic acid may be isononanoic acid comprising a mixture of 3,3,5-trimethyl-hexanoic acid, 2,5,5-trimethyl-hexanoic acid and 7-methyl-octanoic acid or neodecanoic acid comprising a mixture of 7,7-dimethyloctanoic acid, 2,2,3,5-tetramethyl-hexanoic acid, 2,4-dimethyl-2-isopropylpentanoic acid and 2,5-dimethyl-2-ethylhexanoic acid. Particularly preferably, the carboxylic acid is n-hexanoic acid or n-heptanoic acid.

After replacing the solvent by the carboxylic acid, the obtained carboxylic acid-wet DCDPSO is used in the reaction (VI) to obtain a reaction mixture comprising DCDPS and carboxylic acid.

The reaction (VI) of DCDPSO and the oxidizing agent in a carboxylic acid as solvent in principle can be operated as known by a skilled person from WO-A 2018/007481.

It is preferred that in the reaction (VI) for producing DCDPS using the DCDPSO, generally a solution is used comprising DCDPSO and carboxylic acid in a weight ratio of DCDPSO to carboxylic acid in a range from 1 : 2 to 1 : 6, more preferred in a range from 1 : 2 to 1 : 4, particularly in a range from 1 : 2,5 to 1 : 3,5. To achieve this ratio, additional carboxylic acid can be added to the carboxylic acid-wet DCDPSO. By such a ratio of DCDPSO to carboxylic acid, the solubility of DCDPS produced by oxidation of the DCDPSO is at an optimum at the temperature of the oxidization reaction and of a subsequent crystallization process for obtaining crystallized DCDPS. Such a ratio particularly allows a sufficient heat dissipation in the reaction and an amount of DCDPS in the mother liquor obtained by crystallization which is as low as possible.

Further it is preferred that the reaction comprises producing DCDPS by reacting the solution comprising DCDPSO in the carboxylic acid with an oxidizing agent to obtain a reaction mixture comprising DCDPS and carboxylic acid, wherein the concentration of water in the reaction mixture is kept below 5 wt%. By keeping the concentration of water below 5 wt% it is possible to use a linear C₆-C₁₀ carboxylic acid which is particularly preferred for only slightly health hazard-ousness and a good biodegradability. Another advantage of using a linear C₆-C₁₀ carboxylic acid is that the linear C₆-C₁₀ carboxylic acid shows a good separability from water at low temperatures which allows separation of the linear C₆-C₁₀ carboxylic acid without damaging the product and which further allows recycling the linear C₆-C₁₀ carboxylic acid as solvent into the oxidation process.

For carrying out the reaction, the solution preferably is provided in a reactor. This reactor can be any reactor which allows mixing and reacting of the components fed into the reactor. A suitable reactor for example is a stirred tank reactor or a reactor with forced circulation, particularly a reactor with external circulation and a nozzle to feed the circulating liquid. For reasons of process stability and process reliability, it is preferred that the reactor is a stirred tank reactor with an axially conveying stirrer.

For controlling the temperature in the reactor, it is further preferred to use a reactor with heat exchange equipment, for example a double jacket or a heating coil. This allows additional heating or heat dissipation during the reaction and keep the temperature constant or in a predefined temperature range at which the reaction is carried out. Preferably, the reaction temperature is kept from 70 to 110°C, more preferred from 80 to 100°C, particularly from 85 to 95°C, for example 86, 87, 88, 89 90, 91, 92, 93, 94°C.

To obtain DCDPS, the solution comprising DCDPSO and carboxylic acid is oxidized by an oxidizing agent. Therefore, the oxidizing agent preferably is added to the solution to obtain a reaction mixture. From the reaction mixture the residual moisture comprising DCDPS can be obtained.

The oxidizing agent used for oxidizing DCDPSO for obtaining DCDPS preferably is at least one peroxide. Preferably, the reaction (VI) is carried out in the presence of one or two, particularly in the presence of one peracid. The at least one peracid may be a linear or branched C₁ to C₁₀ peracid, which may be unsubstituted or substituted, e.g. by linear or branched C₁ to C₅ alkyl or halogen, such as fluorine. Examples thereof are peracetic acid, performic acid, perpropionic acid, percaprionic acid, pervaleric acid or pertrifluoroacetic acid. Particularly preferably the at least one peracid is a C₆ to C₁₀ peracid, for example 2-ethylhexanoic peracid. If the at least one peracid is soluble in water, it is advantageous to add the at least one peracid as aqueous solution. Further, if the at least one peracid is not sufficiently soluble in water, it is advantageous that the at least one peracid is dissolved in the respective carboxylic acid. Most preferably, the at least one peracid is a linear or branched C₆ to C₁₀ peracid which is generated in situ. Particularly the at least one peracid is generated in situ from at least one carboxylic acid that corresponds to the carboxylic acid used for washing the DCDPSO and which is detailed above.

Particularly preferably, the peracid is generated in situ by using hydrogen peroxide (H₂O₂) as oxidizing agent. At least a part of the added H₂O₂ reacts with the carboxylic acid forming the peracid. The H₂O₂ preferably is added as an aqueous solution, for instance of 1 to 90 wt% solution, such as a 20, 30, 40, 50, 60 70 or 80 wt% solution, preferably as 30 to 85 wt% solution, particularly as a 50 to 85 wt% solution, each being based on the total amount of the aqueous solution. Using a highly concentrated aqueous solution of H₂O₂, particularly a solution of 70 to 85 wt%, for example of 70 wt%, based on the total amount of the aqueous solution, may lead to a reduction of reaction time. It may also facilitate recycling of the at least one carboxylic acid.

Particularly preferably, the at least one peracid is a linear C₆ or C₇ peracid which is generated in situ. To additionally reduce the reaction time and to add only a small amount of water to the reaction mixture, it is particularly preferred that the C₆-C₁₀ carboxylic acid is n-hexanoic acid or n-heptanoic acid and the hydrogen peroxide is a 10 to 85 wt% solution.

To avoid accumulation of the oxidizing agent and to achieve a constant oxidation of the DCDPSO, it is preferred to add the oxidizing agent continuously with a controlled feed rate, for example with a feed rate from 0.002 to 0.01 mol per mol DCDPSO and minute. More preferred, the oxidizing agent is added with a feed rate from 0.003 to 0.008 mol per mol DCDPSO and minute, particularly with a feed rate from 0.004 to 0.007 mol per mol DCDPSO and minute.

If the oxidizing agent is fed in at least two steps, it is preferred to add the oxidizing agent in two steps, wherein adding the oxidizing agent (VI.b) comprises:
(VI.b.1) adding 0.9 to 1.05 mol oxidizing agent per mol DCDPSO uniformly distributed to the solution at a temperature from 70 to 110°C preferably over a period from 1,5 to 5 h in a first step to obtain a reaction mixture;
(VI.b.2) agitating the reaction mixture after completion of the first step at the temperature of the first step preferably for 5 to 30 min without adding oxidizing agent;
(VI.b.3) adding 0.05 to 0.2 mol oxidizing agent per mol DCDPSO to the reaction mixture at a temperature from 80 to 110°C preferably over a period of less than 40 min in a second step;
(VI.b.4) agitating the reaction mixture after completion of the second step at the temperature of the second step preferably for 10 to 30 min without adding oxidizing agent,
(VI.b.5) heating the reaction mixture to a temperature from 95 to 110°C and hold this temperature preferably for 10 to 90 min to obtain a reaction mixture comprising DCDPS.

If the oxidation of DCDPSO is carried out in at least two steps, for converting the DCDPSO into DCDPS, the DCDPSO is oxidized by adding the oxidizing agent in the first and second steps to the solution comprising DCDPSO and carboxylic acid.

"Uniformly distributed" in this context means that the oxidizing agent can be added either continuously at a constant feed rate or at periodically changing feed rates. Besides continuous periodically changing feed rates, periodically changing feed rates also comprise discontinuously changing periodical feed rates for example feed rates where oxidizing agent is added for a defined time, then no oxidizing agent is added for a defined time and this adding and not adding is repeated until the complete amount of oxidizing agent for the first step is added. The period in which the oxidizing agent is added, preferably is from 1,5 to 5 h, more preferred from 2 to 4 h, particularly from 2,5 to 3,5 h. By adding the oxidizing agent uniformly distributed over such a period, it can be avoided that oxidizing agent accumulates in the reaction mixture which may result in an explosive mixture. Additionally, by adding the oxidizing agent over such a period, the process can be scaled up in an easy way as this allows also in an upscaled process to dissipate the heat from the process. On the other hand, by such an amount decomposition of the hydrogen peroxide is avoided and thus the amount of hydrogen peroxide used in the process can be minimized.

The temperature at which the first step (VI.b.1) is carried out is from 70 to 110°C, preferably from 85 to 100 °C, particularly from 90 to 95 °C. In this temperature range, a high reaction velocity can be achieved at high solubility of the DCDPSO in the carboxylic acid. This allows to minimize the amount of carboxylic acid and by this a controlled reaction can be achieved.

After the addition of the oxidizing agent in the first step (VI.b.1) is completed, the reaction mixture is agitated at the temperature of the first step preferably for 5 to 30 min without adding oxidizing agent (VI.b.2). By agitating the reaction mixture after completion of adding the oxidizing agent, oxidizing agent and DCDPSO which did not yet react are brought into contact to continue the reaction forming DCDPS for reducing the amount of DCDPSO remaining as impurity in the reaction mixture.

To further reduce the amount of DCDPSO in the reaction mixture, after completing of agitating without adding oxidizing agent, from 0.05 to 0.2 mol oxidizing agent per DCDPSO, preferably from 0.06 to 0.15 mol oxidizing agent per mol DCDPSO, particularly from 0.08 to 0.1 mol oxidizing agent per mol DCDPSO are added to the reaction mixture in the second step (VI.b.3).

In the second step (VI.b.3), the oxidizing agent preferably is added in a period from 1 to 40 min, more preferred in a period from 5 to 25 min, particularly in a period from 8 to 15 min. The addition of the oxidizing agent in the second step may take place in the same way as in the first step. Further, it is also possible to add the entire oxidizing agent of the second step at once.

The temperature of the second step (VI.b.3) is from 80 to 110°C, more preferred from 85 to 100 °C, particularly from 93 to 98°C. It further is preferred that the temperature in the second step is from 3 to 10°C higher than the temperature in the first step. More preferred the temperature in the second step is from 4 to 8°C higher than the temperature in the first step, particularly preferably, the temperature in the second step is from 5 to 7°C higher than the temperature in the first step. By the higher temperature in the second step, it is possible to achieve a higher reaction velocity.

After addition of the oxidizing agent in the second step, the reaction mixture is agitated at the temperature of the second step preferably for 10 to 20 min to continue the oxidation reaction of DCDPSO forming DCDPS in (VI.b.4).

To complete the oxidation reaction, after agitating at the temperature of the second step without adding oxidizing agent, the reaction mixture is heated to a temperature from 95 to 110°C, more preferred from 95 to 105°C, particularly from 98 to 103°C and held at this temperature preferably for 10 to 90 min, more preferred from 10 to 60 min, particularly from 10 to 30 min in (VI.b.5). In the oxidizing process, particularly when using H₂O₂ as oxidizing agent, water is formed. Further, water may be added with the oxidizing agent. Preferably, the concentration of the water in the reaction mixture is kept below 5 wt%, more preferred below 3 wt%, particularly below 2 wt%. By using aqueous hydrogen peroxide with a concentration of from 70 to 85 wt% the concentration of water during the oxidization reaction is kept low. It even may be possible to keep the concentration of water in the reaction mixture during the oxidization reaction below 5 wt% without removing water by using aqueous hydrogen peroxide with a concentration of from 70 to 85 wt%.

Additionally or alternatively, it may be necessary to remove water from the process for keeping the concentration of water in the reaction mixture below 5 wt%. To remove the water from the process, it is for example possible to strip water from the reaction mixture. Stripping thereby preferably is carried out by using an inert gas as stripping medium. If the concentration of water in the reaction mixture remains below 5 wt% when using aqueous hydrogen peroxide with a concentration of from 70 to 85 wt% it is not necessary to additionally strip water. However, even in this case it is possible to strip water to further reduce the concentration.

The amount of inert gas used for stripping the water preferably is from 0 to 2 Nm³/h/kg, more preferably from 0.2 to 1.5 Nm³/h/kg, particularly from 0.3 to 1 Nm³/h/kg. The gas rate in Nm³/h/kg can be determined according to DIN 1343, January 1990 as relative gas flow. Stripping of water with the inert gas may take place during the whole process or during at least one part of the process, whereby between the parts stripping of water may be interrupted, independent of the mode in which the oxidizing agent is added. Particularly preferably, the water is stripped by continuously bubbling an inert gas into the reaction mixture.

To avoid different conversion rates of DCDPSO it is preferred to homogenize the reaction mixture. For reasons of process stability and process reliability, it is preferred to therefore employ a stirred tank reactor with an axially conveying stirrer. To support the oxidation reaction, it is further advantageous to additionally add at least one acidic catalyst to the reaction mixture. An additional acid in this context is an acid which is not the carboxylic acid which serves as solvent. The additional acid may be an inorganic or organic acid, with the additional acid preferably being an at least one strong acid. Preferably, the strong acid has a pKₐ value from -9 to 3, for instance -7 to 3 in water. It is more preferred that the at least one strong acid has a negative pKₐ value, such as from -9 to -1 or -7 to -1 in water.

Examples for inorganic acids being the at least one strong acid are nitric acid, hydrochloric acid, hydrobromic acid, perchloric acid, and/or sulfuric acid. Particularly preferably, one strong inorganic acid is used, in particular sulfuric acid. While it may be possible to use the at least one strong inorganic acid as aqueous solution, it is preferred that the at least one inorganic acid is used neat. Suitable strong organic acids for example are organic sulfonic acids, whereby it is possible that at least one aliphatic or at least one aromatic sulfonic acid or a mixture thereof is used. Examples for the at least one strong organic acid are para-toluene sulfonic acid, methane sulfonic acid or trifluormethane sulfonic acid. Particularly preferably the strong organic acid is methane sulfonic acid. Besides using either at least one inorganic strong acid or at least one organic strong acid, it is also possible to use a mixture of at least one inorganic strong acid and at least one organic strong acid as acidic catalyst. Such a mixture for example may comprise sulfuric acid and methane sulfonic acid.

The acidic catalyst preferably is added in catalytic amounts. Thus, the amount of acidic catalyst used may be from 0.1 to 0.3 mol per mol DCDPSO, more preferred from 0.15 to 0.25 mol per mol DCDPSO. However, it is preferred to employ the acidic catalyst in an amount of less than 0.1 mol per mol DCDPSO, such as in an amount from 0.001 to 0.08 mol per mol DCDPSO, for example from 0.001 to 0.03 mol per mol DCDPSO. Particularly preferably, the acidic catalyst is used in an amount from 0.005 to 0.01 mol per mol DCDPSO.

The oxidation reaction can be carried out at atmospheric pressure or at a pressure which is below or above atmospheric pressure, for example from 10 to 900 mbar(abs). Preferably, the oxidation reaction is carried out at a pressure from 200 to 800 mbar(abs), particularly from 350 to 700 mbar(abs), such as 400, 500 or 600 mbar(abs). Surprisingly, the reduced pressure has the additional advantage that the total conversion of DCDPS can be increased and thus a very low content of remaining DCDPS in the product can be achieved.

The oxidization reaction can be carried out under ambient atmosphere or inert atmosphere. If the oxidization reaction is carried out under inert atmosphere, it is preferred to purge the reactor with an inert gas before feeding the DCDPSO and the carboxylic acid. If the oxidization reaction is carried out under an inert atmosphere and the water formed during the oxidation reaction is stripped with an inert gas, it is further preferred that the inert gas used for providing the inert atmosphere and the inert gas which is used for stripping the water is the same. It is a further advantage of using an inert atmosphere that the partial pressure of the components in the oxidization reaction, particularly the partial pressure of water is reduced.

To obtain the DCDPS as product, the reaction mixture is separated into a residual moisture comprising DCDPS (in the following also termed as "moist DCDPS") and a liquid phase comprising the carboxylic acid in (VII).

If the moisture in the moist DCDPS does not have a negative effect on processes which use the DCDPS, the moist DCDPS can be withdrawn from the process as a crude product. However, it is preferred to further work-up the moist DCDPS.

The separation can be carried out by any known process, for example by a distillation or by cooling to form a suspension and subsequent solid-liquid separation of the suspension. Particularly preferably, the reaction mixture is separated by cooling and subsequent solid-liquid separation.

Preferably, for separating the reaction mixture into the moist DCDPS and the liquid phase comprising the carboxylic acid, the reaction mixture is cooled to a temperature below the saturation point of DCDPS to obtain a suspension comprising crystallized DCDPS and a liquid phase. The suspension is separated by a solid-liquid separation into moist DCDPS and a second mother liquor. The solid-liquid separation thereby can be carried out by any suitable separation means for example by filtration or centrifugation.

The cooling for crystallizing DCDPS can be carried out in any crystallization apparatus or any other apparatus which allows cooling of the organic mixture, for example an apparatus with surfaces that can be cooled such as a vessel or tank with cooling jacket, cooling coils or cooled baffles like so-called "power baffles".

To avoid precipitation and fouling on cooled surfaces, it is particularly preferred that separating the reaction mixture in (VII) comprises:
(VII.a) mixing the reaction mixture with water in a gastight closed vessel to obtain a liquid mixture;
(VII.b) cooling the liquid mixture obtained in (VII.a) to a temperature below the saturation point of 4,4'-dichlorodiphenyl sulfone by
   (i) reducing the pressure in the gastight closed vessel to a pressure at which the water starts to evaporate,
   (ii) condensing the evaporated water by cooling
   (iii) mixing the condensed water into the liquid mixture in the gastight closed vessel,
      to obtain a suspension comprising crystallized 4,4'-dichlorodiphenyl sulfone;
(Vll.c) carrying out a solid-liquid-separation of the suspension to obtain the moist DCDPS and the liquid phase comprising the carboxylic acid.

This process allows for cooling the DCDPS comprising reaction mixture without cooling surfaces onto which particularly at starting the cooling process crystallized DCDPS accumulates and forms a solid layer. This enhances the efficiency of the cooling process. Also, additional efforts to remove this solid layer can be avoided.

If cooling is performed according to (VII.b), the suspension which is subjected to the solid-liquid separation additionally contains water besides the crystallized DCDPS and the carboxylic acid.

Particularly when carboxylic acids are used as solvent which have a boiling point above 150°C at 1 bar, cooling by reducing the pressure to evaporate solvent, to condense the evaporated solvent by cooling and recycling the condensed solvent back into the gastight vessel would require a high energy consumption to achieve the necessary low pressures. By mixing the reaction mixture with water and to evaporate, condense and recycle the condensed water, it is possible to shift the saturation point by cooling without evaporating solvent at high temperatures or to reduce the pressure to very low values which is very energy consuming. Thus, particularly no change in color of the DCDPS occurs. Surprisingly, cooling and crystallization of DCDPS by adding water, reducing the pressure to evaporate water, condensing the water by cooling and recycle the condensed water and mix it into the reaction mixture even can be carried out when carboxylic acids are used as solvent which have a poor solubility in water.

To crystallize the DCDPS, it is preferred to provide crystal nuclei. The crystal nuclei can be provided in the same way as described above for the crystallization of DCDPSO. Preferably, the crystal nuclei are generated in situ in an initializing step. The initializing step preferably comprises following steps before reducing pressure in step (i):
- reducing the pressure in the gastight closed vessel such that the boiling point of the water in the liquid mixture is from 80 to 95°C;
- evaporating water until an initial formation of solids takes place;
- increasing the pressure in the vessel and heating the liquid mixture in the gastight closed vessel to a temperature from 1 to 10°C below the saturation point of DCDPS.

For generating the crystal nuclei in the initializing step, it is possible to only evaporate water until an initial formation of solids take place. It is also possible to entirely condense the evaporated water by cooling and to return all the condensed water into the gastight closed vessel. The latter has the effect that the organic mixture in the gastight closed vessel is cooled and solid forms. A mixture of both approaches, where only a part of the evaporated and condensed water is returned into the gastight vessel, is also viable.

Preferably, the pressure reduction (VII.b(i)) is temperature controlled with a stepwise cooling profile from 5 to 25 K/h to approximate a constant supersaturation with increasing solid content and thus, more crystalline surface for growth.

The crystallization preferably is continued until the solids content in the suspension in the last step of the crystallization is from 5 to 50 wt%, more preferred from 5 to 40 wt%, particularly from 20 to 40 wt%, based on the mass of the suspension.

To achieve this solids content in the suspension, it is preferred to reduce the pressure in step (i) until the suspension which is obtained by the cooling has cooled down to a temperature from 10 to 30°C, more preferred from 15 to 30°C, particularly from 20 to 30°C.

The pressure at which this temperature is achieved depends on the amount of water in the liquid mixture. Preferably, the amount of water mixed to the liquid mixture is such that the amount of water in the liquid mixture is from 10 to 60 wt% based on the total amount of the liquid mixture. More preferred, the amount of water mixed to the liquid mixture is such that the amount of water in the liquid mixture is from 10 to 50 wt% based on the total amount of the liquid mixture and, particularly, the amount of water mixed to the liquid mixture is such that the amount of water in the liquid mixture is from 15 to 35 wt% based on the total amount of the liquid mixture.

To support cooling of the organic mixture it is further possible to provide the gastight closed vessel with coolable surfaces for an additional cooling. The coolable surfaces for example can be a cooling jacket, cooling coils or cooled baffles like so called "power baffles". Surprisingly, forming of precipitations and fouling on coolable surfaces can be avoided or at least considerably reduced, if the additional cooling is started not before the temperature of the liquid mixture is reduced to a temperature from 20 to 60°C, more preferred from 20 to 50°C, particularly from 20 to 40°C.

After completing the cooling and crystallization by pressure reduction, the process is finished and preferably the pressure is set to ambient pressure, again. After reaching ambient pressure, the suspension which formed by cooling the liquid mixture in the gastight closed vessel is subjected to the solid-liquid separation (VII.c). In the solid liquid separation process, the crystallized DCDPS formed by cooling is separated from the carboxylic acid and the water.

The solid-liquid-separation for example comprises a filtration, centrifugation or sedimentation. Preferably, the solid-liquid-separation is a filtration. In the solid-liquid-separation liquid the second mother liquor comprising carboxylic acid and water is removed from the solid DCDPS and moist DCDPS is obtained as product. If the solid-liquid-separation is a filtration, the moist DCDPS is called "DCDPS filter cake".

The solid-liquid-separation preferably is carried out in the same way as the solid-liquid-separation of the DCDPSO comprising suspension.

To purify the moist DCDPS, the moist DCDPS preferably is washed with an aqueous base in a first phase and subsequently with water in a second phase. By washing, particularly remainders of the carboxylic acid and further impurities, for example undesired by-products which formed during the process for producing the DCDPS are removed.

By adding a strong acid to the aqueous base after washing the carboxylic acid can be reused and due to reduced total organic carbon (TOC) the aqueous phase is easier to dispose. The aqueous base used for washing the moist DCDPS can be one aqueous base or a mixture of at least two aqueous bases. Preferably, the aqueous base used for washing in the first phase preferably is an aqueous alkali metal hydroxide or a mixture of at least two aqueous alkali metal hydroxides, for example aqueous potassium hydroxide or sodium hydroxide, particularly sodium hydroxide. If an alkali metal hydroxide is used as aqueous base, the aqueous alkali metal hydroxide preferably comprises from 1 to 50 wt% alkali metal hydroxide based on the total amount of aqueous alkali metal hydroxide, more preferred from 1 to 20 wt% alkali metal hydroxide based on the total amount of aqueous alkali metal hydroxide, particularly from 2 to 10 wt% alkali metal hydroxide based on the total amount of aqueous alkali metal hydroxide. This amount is sufficient for properly washing the moist DCDPS.

By using the aqueous alkali metal hydroxide, the anion of the carboxylic acid reacts with the alkali metal cation of the alkali metal hydroxide forming an organic salt and water, which is soluble in water. Therefore, washing the moist DCDPS with water allows to achieve DCDPS as product which contains less than 1 wt%, preferably less than 0.7 wt%, particularly less than 0.5 wt% organic impurities.

For obtaining DCDPS with such a small content of organic impurities, the amount of the aqueous base, particularly the alkali metal hydroxide used for the washing in the first phase preferably is from 0.5 to 10 kg per kg dry DCDPS, more preferred from 1 to 6 kg per kg dry DCDPS, particularly from 2 to 5 kg per kg dry DCDPS.

As the water of the aqueous base and the water produced by the reaction of the anion of the base with the carboxylic acid generally is not sufficient to remove all of the organic salt and as further part of the aqueous base may stay in the moist DCDPS, the moist DCDPS is washed with water in the second phase. By washing with water, remainders of the organic salt and of the aqueous base which did not react are removed. Removal can be monitored by measuring the pH of the moist DCDPS. The water then can be easily removed from the DCDPS by usual drying processes known to a skilled person to obtain dry DCDPS as product. Alternatively, it is possible to use the water wet DCDPS which is obtained after washing with water in subsequent process steps.

The washing with water in the second phase preferably is carried out in two washing steps. In this case, it is particularly preferred to use fresh water for the washing in the second washing step and to use the water which has been used in the second washing step in the first washing step. This allows the amount of water which is used for washing in total to be kept low.

Washing of the moist DCDPS preferably is operated at ambient temperature. It is also possible to wash the moist DCDPS at temperatures different to ambient temperature, for instance above ambient temperature.

Particularly the aqueous base which was used for washing the moist DCDPS contains either carboxylic acid or the organic salt of the carboxylic acid. To reduce the amount of carboxylic acid which is withdrawn with the water and subjected to purification in a purification plant and thereby completely removed, according to the invention, the aqueous base is mixed with a strong acid after being used for washing. The strong acid preferably is selected such that the second salt which forms from the anion of the strong acid has a good solubility in water and a poor solubility in the carboxylic acid. In this context "good solubility" means at least 20 g per 100 g solvent can be dissolved and "poor solubility" means that less than 5 g per 100 g solvent can be dissolved in the solvent.

The poor solubility of the second salt in the carboxylic acid has the effect that the carboxylic acid which can be recovered comprises less than 3 ppm wt% impurities based on the total mass of the carboxylic acid. This allows further use of the carboxylic acid without further purification steps.

Depending on the aqueous base which is used for the washing of the moist DCDPS, the strong acid preferably is sulfuric acid or a sulfonic acid, like paratoluene sulfonic acid or alkane sulfonic acid, for example methane sulfonic acid. If the aqueous base is an alkali metal hydroxide, the strong acid particularly preferably is sulfuric acid. Particularly if a strong acid is used as acidic catalyst in the reaction, the strong acid which is used for mixing with the aqueous base to remove the carboxylic acid and the acid used as acidic catalyst preferably are the same.

To allow reusing the carboxylic acid, the carboxylic acid has to be separated from the aqueous phase. This preferably is carried out by a phase separation. The carboxylic acid separated by the phase separation can be used in any process in which a respective carboxylic acid is used. However, it is particularly preferred to recycle the carboxylic acid into the process for producing the DCDPS, for example by using in the washing (V) or as solvent in (VI). If the carboxylic acid contains impurities after being separated off by the phase separation, it is further possible, to subject the carboxylic acid to additional purifying steps like washing or distillation to remove high boiling or low boiling impurities.

Due to the comparatively small amount of carboxylic acid in the aqueous base after being mixed with the strong acid, it is possible to add at least a part of the second mother liquor to the aqueous base mixed with the strong acid or to mix the aqueous base after being used with at least a part of the second mother liquor and the strong acid before carrying out the phase separation. This allows to improve the efficiency of the phase separation.

Particularly in case the cooling and crystallization is carried out in the gastight closed vessel by adding water and reducing the pressure, the second mother liquor additionally contains water. To allow reuse of the carboxylic acid in this case also the filtrate must be subject to a phase separation. Mixing the aqueous base mixed with the strong acid and the second mother liquor in this case has the additional advantage that only one phase separation has to be carried out for separating the organic carboxylic acid from the aqueous phase.

Depending on the amounts of organic phase and aqueous phase and the process used for phase separation, it may be necessary to increase the amount of the aqueous phase in the mixture. This can be achieved for example by circulating at least a part of the aqueous phase through the phase separation apparatus and the mixing device.

To avoid particle clogging, after filtration the second mother liquor can be used for flushing the outlet for the aqueous base of the filter.

Besides feeding the part of the aqueous phase which was branched off for circulating into the phase separation apparatus before or after mixing with the second mother liquor and the aqueous base after being mixed with the strong acid, it is also possible to recirculate the part of the aqueous phase into the mixing of the aqueous base with the strong acid.

Additionally or alternatively, it is also possible to increase the amount of the aqueous phase by feeding at least a part of the water which was used for washing in the second phase after the washing with the aqueous base, into the phase separation. By feeding at least a part of this water into the phase separation even traces of organic impurities, particularly carboxylic acid which may still be comprised in the DCDPS after washing with the aqueous base can be regained.

In an alternative, it is also possible to mix the second mother liquor and the organic phase obtained in the phase separation. In this case, the second mother liquor may undergo a phase separation to remove water from the second mother liquor before mixing, but it is also possible to mix the second mother liquor with the organic phase without subjecting the second mother liquor to any further process steps before mixing with the organic phase.

To allow reusing the liquid phase comprising carboxylic acid which is obtained by separating the reaction mixture in (VII), the liquid phase preferably is purified by
- distilling a part of the liquid phase comprising carboxylic acid;
- stripping low boilers from at least a part of the liquid phase comprising carboxylic acid; and
- recycling the purified carboxylic acid into the reaction (VI).

This process allows reusing most of the carboxylic acid used as solvent for the reaction and thus to reduce the amount of byproducts which have to be removed and disposed.

To avoid an increase in the amount of impurities contained in the carboxylic acid, the liquid phase comprising the carboxylic acid which is separated off the reaction mixture is subjected to this purifying process. By purifying a large amount of impurities can be removed from the carboxylic acid and thus from the process. Thereby, it is avoided that the produced DCDPS is contaminated with these impurities.

For purifying the liquid phase comprising the carboxylic acid from 2 to 25 vol% of the liquid phase comprising carboxylic acid are subjected to distillation. By distillation, low boiling impurities and high boiling impurities are removed from this part of the liquid phase comprising carboxylic acid. To further remove low boilers from the liquid phase comprising carboxylic acid, at least a part of this liquid phase is stripped with an inert gas. Thereby it is possible, to carry out the distillation of a part of the liquid phase before or after stripping of the whole liquid phase. Further, it is possible to separate the liquid phase into two parts and subject one part to distillation and the other part to stripping.

Independently of being mixed before or after the phase separation, the mixture of organic phase and second mother liquor or, alternatively, the organic phase is the liquid phase comprising carboxylic acid which is purified.

In contrast to a distillation, by stripping also small amounts of the low boilers can be removed. This allows to achieve a purified carboxylic acid comprising less than 1.5 wt%, preferably less than 1 wt%, particularly less than 0.6 wt% of water and less than 1.5 wt%, preferably less than 1 wt%, particularly less than 0.6 wt% of monochlorobenzene, each based on the total amount of the purified carboxylic acid recycled into the reaction (VI).

Independently of whether the liquid phase comprising carboxylic acid is subjected to stripping in total or only a part of the liquid phase comprising carboxylic acid is subjected to stripping, stripping preferably is carried out at a temperature from 80 to 100°C, more preferred at a temperature from 85 to 95 °C, particularly at a temperature from 85 to 90°C and a pressure from 0.1 to 0.7 bar(abs), more preferred from 0.2 to 0.4 bar(abs), particularly from 0.25 to 0.35 bar(abs).

In the stripping process, a stripping gas flows through the liquid phase comprising carboxylic acid. The stripping gas is selected such that it is inert towards the components comprised in the liquid phase comprising carboxylic acid. A suitable stripping gas preferably is an inert gas.

Stripping can be carried out in any apparatus suitable for a stripping process and known to a skilled person.

It has been shown that for removing high boiling impurities and low boiling impurities in such an amount that no accumulation occurs, only a part of the crude carboxylic acid must be subjected to the distillation. This has the advantage, that energy can be saved and a smaller apparatus for the distillation can be used.

If the total liquid phase comprising carboxylic acid is stripped and the thus obtained crude carboxylic acid is separated into a first and a second carboxylic acid stream, the second carboxylic acid stream which is fed into the distillation preferably contains from 2 to 25 vol% of the crude carboxylic acid. More preferred, the second carboxylic acid stream contains from 5 to 20 vol%, particularly from 7 to 15 vol% of the crude carboxylic acid and the first carboxylic acid stream the rest of the crude carboxylic acid.

If the liquid phase comprising carboxylic acid is separated into a first part and a second part which is subjected to distillation, the second part which is fed into the distillation comprises from 2 to 25 vol%, more preferred from 5 to 20 vol%, particularly from 7 to 15 vol% of the liquid phase comprising carboxylic acid and the first part the rest of the liquid phase comprising carboxylic acid.

The distillation of the second carboxylic acid stream or the second part of the liquid phase comprising carboxylic acid can be carried out in any apparatus suitable for carrying out a distillation and which allows to withdraw a stream comprising high boilers, a stream comprising low boilers and a stream comprising at least one component having a boiling temperature between the boiling point of high boilers and low boilers.

The distillation preferably is carried out at a bottom temperature in a range from 130 to 250°C, more preferred in a range from 150 to 220°C, particularly in a range from 190 to 215°C, a top temperature from 50 to 150°C, more preferred from 100 to 140°C, particularly in a range from 120 to 140°C, and a pressure in a range from 10 mbar(abs) to 400 mbar(abs), more preferred in a range from 20 mbar(abs) to 300 mbar(abs), particularly in a range from 30 mbar(abs) to 250 mbar(abs).

The carboxylic acid withdrawn from the distillation column as side stream is mixed with the first carboxylic acid stream and recycled into the reaction (VI) or the washing (V).

To keep the reaction temperature constant and further to avoid heating of the components used for the reaction in the reactor, it is preferred to temper the purified carboxylic acid to a temperature from 80 to 100°C before recycling it into the reaction. This temperature corresponds to the temperature at which the reaction is carried out and thus it is not necessary to heat huge amounts of components in the reactor before the reaction starts.

After washing with an aqueous base and subsequently with water, the thus obtained first purified DCDPS still may contain remainders of the carboxylic acid, DCDPSO and isomers. To remove these impurities, it is preferred to subject the first purified DCDPSO to a further purifying process. Alternatively, it is also possible to subject the moist DCDPS which is obtained in separating the reaction mixture (VII) to the following purifying procedure for removing impurities. However, it is particularly preferred to wash the moist DCDPS with the aqueous base and subsequently with water before carrying out the following washing procedure.

For purifying, the moist DCDPS or the first purified DCDPS, if the moist DCDPS is washed, is further worked up by:
(A) dissolving the moist DCDPS or the first purified DCDPS in an organic solvent in which DCDPS has a solubility of from 0.5 to 20% at 20°C to obtain a solution;
(B) cooling the solution to a temperature below the saturation point of DCDPS to obtain a suspension comprising crystallized DCDPS;
(C) carrying out a solid-liquid separation to obtain purified residual moisture comprising DCDPS and a third mother liquor;
(D) washing the purified residual moisture comprising DCDPS with an organic solvent in which DCDPS has a solubility of from 0.5 to 20% at 20°C;
(E) optionally repeating steps (B) to (D);
(F) drying the 4,4'-dichlorodiphenyl sulfone
(G) optionally working up and preferably recycling into the dissolving (A) at least a part of the third mother liquor and optionally the organic solvent in which 4,4'-dichlorodiphenyl sulfone has a solubility of 0.5 to 20% at 20°C used for washing by distillation.

The solubility of DCDPS in the organic solvent is defined as S = (m_{DCDPS} / m_{Solv}) · 100 [%], with m_{DCDPS} being the amount of DCDPS in kg and m_{Solv} being amount of solvent in kg.

By this purification process it is possible to further reduce the impurities in the DCDPS and to achieve a DCDPS which contains less than 0,3 wt% isomers, less than 10 ppm 4,4'-dichlorodiphenyl sulfoxide, particularly less than 2 ppm 4,4'-dichlorodiphenyl sulfoxide and less than 200 ppm, particularly less than 100 ppm carboxylic acid, each based on the total amount of dry DCDPS.

To purify the moist DCDPS or the first purified DCDPS (in the following termed as crude DCDPS), at first the crude DCDPS which may contain residual moisture, for example water from washing the DCDPS after completing the reaction or carboxylic acid which is used as solvent in the reaction, is mixed with the organic solvent in which DCDPS has a solubility of from 0.5 to 20% at 20°C (in the following termed as organic solvent). By mixing the crude DCDPS with the organic solvent, a suspension forms and the crude DCDPS starts to dissolve. If the crude DCDPS does not contain water or if the water content in the crude DCDPS is below 1 wt%, it is preferred to add water to the suspension. If water is added to the suspension, it is possible to use a liquid mixture comprising the organic solvent and water or to add the water separately. The water can be added simultaneously with the organic solvent, before adding the organic solvent or after finishing the addition of the organic solvent. However, particularly preferably, crude DCDPS is used which contains water in an amount from 1 to 30 wt%, more preferred from 2 to 25 wt% and particularly from 3 to 20 wt%.

To support dissolving the crude DCDPS in the organic solvent, the suspension comprising the crude DCDPS and the organic solvent is heated. Preferably, the suspension is heated to a temperature from 90 to 120°C, particularly in a range from 100 to 110°C. To avoid evaporation of the organic solvent during the heating of the suspension, the heating preferably is carried out at elevated pressure. Preferably, during heating for supporting dissolving the crude DCDPS in the organic solvent, the pressure is set to 2 to 10 bar(abs), more preferred to 3 to 5 bar(abs), particularly to 3.5 to 4.5 bar(abs).

After completing dissolving the DCDPS in the organic solvent, the thus produced suspension is cooled to a temperature below the saturation point of DCDPS to obtain a suspension comprising crystallized DCDPS. Due to cooling the suspension, the DCDPS starts to crystallize again. This new crystallization of the DCDPS in the organic solvent has the advantage, that impurities which may have been comprised in the crude DCDPS remain solved in the organic solvent and the crystals newly formed by cooling have a higher purity. Dissolving the DCDPS in the organic solvent to obtain the suspension is completed when at least 90% of the DCDPS are dissolved. Particularly preferably, dissolving the DCDPS in the organic solvent is completed when all of the DCDPS is dissolved.

To avoid a too fast crystal growth by which impurities solved in the organic solvent would be incorporated into the newly formed crystals, it is preferred to cool the solution in (B) with a multi-step cooling rate of initially from 3 to 15 K/h for 0,5 to 3h more preferred from 0,5 to 2h and later on from 10 to 40 K/h, more preferred with a cooling rate from 15 to 30 K/h, particularly with a cooling rate from 18 to 25 K/h until a predefined end temperature is reached. Besides the preferred multi-step cooling, a one-step cooling with a cooling rate from 10 to 30 K/h until the end temperature is reached, also is possible.

The lower the temperature to which the solution is cooled, the lower is the amount of DCDPS still solved in the third mother liquor. On the other hand, the efforts needed for cooling increase with decreasing temperature. Therefore, the solution preferably is cooled in (B) to a temperature from -10 to 25°C, more preferred to a temperature from 0 to 20°C, particularly to a temperature from 3 to 12°C. Cooling to a temperature in such a range has the advantage that the stage yield in regard to the necessary efforts is optimized. This has the additional effect that waste streams of the overall process can be minimized.

Cooling of the solution for crystallizing DCDPS preferably is carried out in the same mode as described above for crystallizing DCDPSO and preferably comprises:
(B.i) reducing the pressure of the solution to a pressure at which the organic solvent starts to evaporate;
(B.ii) condensing the evaporated organic solvent by cooling;
(B.iii) mixing the condensed organic solvent with the solution to obtain the suspension.

If the crude DCDPS contains water, in this process for cooling in a gastight closed vessel it cannot be excluded that besides the organic solvent also a part of the water evaporates. Therefore, when the term "organic solvent" is used in the description of evaporation steps and condensation steps in the cooling process (B), the skilled person appreciates that the organic solvent also may comprise water.

It is particularly preferred to reduce the pressure in step (B.i) until the temperature in the gastight closed vessel reaches the predefined value from -10 to 25°C, preferably from 0 to 20°C, particularly from 3 to 12°C. At these predefined temperatures the pressure in the gastight closed vessel typically is from 10 to 400 mbar(abs), preferably from 10 to 200 mbar(abs), particularly from 30 to 80 mbar(abs). After the predefined temperature value is reached, pressure reduction is stopped and then the gastight closed vessel is vented until ambient pressure is reached. The temperature profile in the gastight closed vessel preferably is selected such that the solution is subjected to a constant supersaturation.

After reaching ambient pressure the suspension comprising particulate 4,4'-dichlorodiphenyl sulfone in the organic solvent (in the following termed as "suspension") which formed in the gastight closed vessel by the cooling is withdrawn and fed into the solid-liquid-separation (C).

Crystallization preferably is continued until the solids content in the suspension in the last step of the crystallization is from 5 to 50 wt%, more preferred from 5 to 40 wt%, particularly from 15 to 40 wt%, based on the mass of the suspension.

Batchwise cooling and crystallization allows a higher flexibility in terms of operating window and crystallization conditions and is more robust against variations in process conditions and is thus usually preferred.

The solid-liquid-separation for example comprises a filtration, centrifugation or sedimentation. Preferably, the solid-liquid-separation is a filtration. In the solid-liquid-separation the third mother liquor is removed from the solid DCDPS and residual moisture comprising purified DCDPS (in the following also termed as "purified moist DCDPS") is obtained. If the solid-liquid-separation is a filtration, the purified moist DCDPS is called "purified filter cake".

Independently of carried out continuously or batchwise, the solid-liquid-separation preferably is performed at ambient temperature or temperatures below ambient temperature, preferably at a temperature from 0 to 10°C.

Apart from the preferred lower temperature, the solid-liquid-separation preferably is carried out in the same way as described above for the solid-liquid-separation of the DCDPSO comprising suspension.

To further purify the DCDPS and to remove impurities from the surface of the crystallized DCDPS and which may be contained in the remaining organic solvent in the purified moist DCDPS as well as non-crystallized DCDPS, the purified moist DCDPS is washed with an organic solvent in which DCDPS has a solubility of from 0.5 to 20% at 20°C (in the following also termed as organic solvent).

The amount of organic solvent used for washing preferably is chosen such that the impurities and the non-crystallized DCDPS are removed from the moist DCDPS. Preferably, the amount of organic solvent used for washing is from 0.3 to 3 kg per kg moist DCDPS, more preferred from 0.5 to 2 kg per kg moist DCDPS, particularly from 0.8 to 1.5 kg per kg moist DCDPS. The lower the amount of organic solvent for washing, the lower are the efforts for recycling the organic solvent and to reuse it in the process cycle, but the less the amount of organic solvent, the less is the washing efficiency regarding carboxylic acids and remaining 4,4'-dichlorodiphenyl sulfoxide and isomers of 4,4'-dichlorodiphenyl sulfone.

After washing, the purified filter cake is removed and dried to obtain dry DCDPS as product.

Washing of the purified moist DCDPS preferably is operated at ambient temperature. It is also possible to wash the purified moist DCDPS at temperatures different to ambient temperature, for instance above ambient temperature.

The third mother liquor obtained in the solid-liquid separation and the organic solvent used for washing still may contain non-crystallized DCDPS. To increase the yield of purified DCDPS in the process and to reduce the amount of organic solvent to be disposed, preferably, at least a part of the third mother liquor and optionally the organic solvent used for washing are worked up by distillation.

By working up at least a part of the third mother liquor and optionally the organic solvent used for washing, it is possible to withdraw at least a part of the DCDPS still solved in the organic solvent as a high boiler and to recycle at least a part of the high boilers either into the purifying process (A) to (E) or into a process step upstream the purifying process to obtain the DCDPS as product and to increase the yield. Further, the organic solvent which is purified in the distillation and obtained as low boiler can be recycled into the purifying step either as organic solvent for solving the DCDPS or as organic solvent for washing the DCDPS. If the organic solvent is used for washing the DCDPS, it must fulfil predefined purity requirements. For being used for washing, the organic solvent preferably contains less than 0.05 wt% impurities, more preferred less than 0.03 wt% impurities, particularly less than 0.015 wt% impurities, each based on the total mass of the organic solvent.

Particularly preferably, the amount of third mother liquor and optionally organic solvent used for washing which is worked up by distillation is from 50 to 100 wt%, more preferred from 70 to 100 wt%, particularly from 90 to 100 wt%, each based on the total amount of third mother liquor and organic solvent used for washing.

The organic solvent which is used for dissolving the DCDPS and the organic solvent which is used for washing the moist DCDPS preferably additionally is chosen such that the solubility of DCDPS at boiling point is up to 100%. Suitable organic solvents for example are symmetric or asymmetric, branched or linear ethers, for example diethyl ether or methyl tert-butyl ether, substituted or unsubstituted aromatic solvents like toluene, monochlorobenzene or benzene, low molecular carboxylic acids, particularly C₁ to C₃ carboxylic acids or low molecular alcohols, particularly C₁ to C₃ alcohols. Preferably, the organic solvent is methanol, ethanol, isopropanol, acetone, methyl tert-butyl ether, acetic acid, toluene, ethyl acetate or monochlorobenzene. Particularly preferably, the organic solvent is a C₁ to C₃ alcohol, particularly methanol, ethanol or isopropanol. Most preferred as organic solvent is methanol.

For dissolving the DCDPS and for washing different organic solvents can be used. However, it is particularly preferred to use the same organic solvent to dissolve the DCDPS for obtaining the solution and for washing the purified moist DCDPS.

If the DCDPS after solid-liquid separation and washing still contains a too large amount of impurities, it is possible to repeat the process steps (A) to (D).

To achieve a dry product, after washing the DCDPS which still contains organic solvent is dried. Drying can be carried out in any dryer which can be used for drying particulate substances.

Drying preferably is carried out using a contact dryer with a wall temperature from 105 to 140°C, more preferred from 110 to 135°C, particularly from 120 to 135°C. By drying the DCDPS at such a temperature, coloring of the DCDPS can be avoided. Drying preferably is continued for 90 to 600 min, more preferred for 180 to 350 min, particularly for 200 to 300 min.

To support the drying process and to avoid damaging the product for example by oxidation, drying in the dryer preferably is carried out in an inert atmosphere. The inert atmosphere is achieved by feeding an inert gas into the dryer.

To allow reusing the organic solvent which is removed from the DCDPS during drying by evaporation, the evaporated organic solvent is condensed by cooling. If an inert gas is fed into the dryer, usually the inert gas and the evaporated organic solvent are withdrawn together from the dryer. In this case, in the condenser the condensed organic solvent is separated from the inert gas. The organic solvent for example can be reused for producing the solution (A) or for washing (D) the DCDPS.

By drying the DCDPS at these conditions, a final product can be achieved which contains less than 400 ppm organic solvent.

After drying, the DCDPS can be cooled down to enable further handling, for example packing in big bags for storage or transport. Suitable coolers for cooling the dried DCDPS can be screw coolers, paddle coolers or other bulk coolers or fluidized bed coolers.

### Examples

### Example 1 (Production of 4,4'-dichlorodiphenyl sulfoxide)

5.5 mol aluminum chloride and 40 mol chlorobenzene were fed into a stirred tank reactor as first reactor. 5 mol thionyl chloride were added to the reaction mixture in 160 min. The reaction in the first reactor was carried out at 10°C. Hydrogen chloride produced in the reaction was withdrawn from the process. After finishing the addition of thionyl chloride the reaction mixture was heated to 60°C.

After finishing the reaction in the first reactor the resulting reaction mixture was fed into a second stirred tank reactor which contained 3400 g hydrochloric acid with a concentration of 11 wt%. The second stirred tank reactor was heated to a temperature of 90°C. After 30 min the mixing was stopped and the mixture separated into an aqueous phase and an organic phase. The aqueous phase was withdrawn and the organic phase was washed with 3000 g water while stirring at 90°C. After washing, stirring was stopped and the mixture separated into an aqueous phase and an organic phase.

The aqueous phase was removed and the organic phase was subjected to a distillation. Monochlorobenzene was distilled from the organic phase until saturation was reached at about 88°C (monitored via a turbidity probe, distillation conditions: 200 mbar(abs)). The organic phase was cooled by reducing the pressure until the temperature reached 30°C.

By the cooling a suspension was obtained containing crystallized DCDPSO. The suspension then was filtrated to obtain a filter cake comprising crystallized DCDPSO, which was washed with 550 g monochlorobenzene.

The combined mother liquor and the monochlorobenzene which was used for washing were subjected to a distillation. In the distillation monochlorobenzene was removed until the amount of combined mother liquor and washing filtrate was reduced to 25 wt%. The distillation was operated at a bottom temperature of 90°C and 200 mbar(abs).

While the distilled monochlorobenzene was reused in the next batch as starting material, 80 wt% of the obtained bottom product were transferred into the crystallization of the next batch.

After washing with monochlorobenzene, the thus obtained monochlorobenzene-wet filter cake comprising crystallized DCDPSO was washed with 300 g n-heptanoic acid and filtrated to obtain n-heptanoic acid wet DCDPSO as filter cake.

The filtrate was subjected to distillation yielding a top fraction of monochlorobenzene and a bottom fraction comprising n-heptanoic acid and DCDPSO. The bottom fraction was topped up with fresh n-heptanoic acid and reused in the next filtration. The distillation was operated at a bottom temperature of 140°C and 100 mbar(abs).

The 4,4'-dichlorodiphenyl sulfoxide yield in the steady state was 1232 g which corresponds to a yield of 91.3%.

The n-heptanoic acid wet DCDPSO had a purity of 89.7 wt%, containing 8.9 wt% n-heptanoic acid, 0.8 wt% monochlorobenzene, 0.3 wt% 4,4'-dichlorodiphenylsulfide and 0.3 wt% 2,4'-di-chlorodiphenylsulfoxide.

### Example 2 (Production of 4,4'-dichlorodiphenyl sulfone)

1113 g of the n-heptanoic acid wet 4,4'-dichlorodiphenyl sulfoxide were dissolved in 2900 g n-heptanoic acid and heated to 90°C. 7.2 g sulfuric acid were added to the solution. Over a period of 3 h and 10 min 143 ml H₂O₂ were added to the solution with a constant feed rate. During the reaction the temperature in the vessel was controlled to 90°C by wall cooling, whereby the temperature in the reactor was determined to be 97 to 99°C. After finishing this step, the reactor was stirred for 15 minutes at a temperature of 97°C. Then, a second amount of 7 ml H₂O₂ was added within 10 minutes. After completing the H₂O₂ dosage the temperature of the solution was raised to 100°C. The reactor was stirred for 20 minutes at a temperature of 100°C.

To the resulting reaction mixture comprising DCDPS and n-heptanoic acid, 881 g water were added with a temperature of 97°C. The thus obtained mixture was cooled by reducing the pressure according to the cooling profile shown in table 1.

**Table 1: cooling profile**

| time ]h] | temperature [°C] | pressure [mbar] |
|---|---|---|
| 0:00 | 97 | 760 |
| 0:50 | 81 | 380 |
| 01:15 | 90 | 580 |
| 1:45 | 90 | 580 |
| 2:45 | 81 | 370 |
| 3:40 | 61,5 | 175 |
| 4:35 | 43 | 70 |
| 6:00 | 18 | 980 |

A suspension comprising 2480 g n-heptanoic acid and DCDPS was obtained by this process.

The suspension then was filtered at ambient temperature to obtain a filter cake comprising about 80 wt% DCDPS, 16 wt% n-heptanoic acid and 4 wt% water. The mother liquor which was separated off the filter cake in the filtration process contained about 78 wt% n-heptanoic acid, 20 wt% water and about 2,5 wt% DCDPS. For filtering the suspension, a glass nutsche was used which was covered with a Sefar^{®} Tetex DLW 17-80000-SK 020 Pharma filter cloth. For filtering, an absolute pressure of 500 mbar was set below the nutsche. After filtration, the filter cake was treated with dry air for 30 s.

### Example 3 (washing the DCDPS with an aqueous base and water)

The filter cake obtained in example 2 then was washed with 2 kg of diluted NaOH 5%. For washing a pressure of 750 mbar(abs) were set to the filtrate side of the nutsche.

Washing with diluted NaOH was followed by washing with 1,5 kg water. For washing with water a pressure of 500 mbar(abs) were set to the filtrate side of the nutsche. Subsequently the filter cake was treated for 30 seconds with dried air.

After washing and treating with dried air, the filter cake contained about 20 wt% water and 0.24 wt% n-heptanoic acid. The final filter cake mass was 1369 g.

The mother liquor obtained in the filtration process was subjected to a phase separation. By phase separation, 482 g aqueous phase and 2712 g organic phase were obtained.

### Example 4 (Purifying of the DCDPS)

500.4 g of the filter cake obtained in example 3 containing 115 g water and containing about 0.24% n-heptanoic acid and about 240 ppm isomers of 4,4'-DCDPS were suspended into 1385 g methanol. This mixture was heated to a temperature of 100°C in a closed vessel. The temperature was kept at 100°C for 2h and 20 min. Then the pressure in the vessel was reduced and methanol started to evaporate. Evaporation of methanol resulted in crystallization of the DCDPS. The temperature in the vessel was reduced linearly with a rate of 10 Kelvin per hour until a temperature of 10°C was reached. After this temperature was reached, the vessel was vented until ambient pressure was achieved. The thus obtained mixture of crystallized DCDPS and methanol was filtered in a filter nutsche. By this filtration a wet filter cake which weighted 613,5g was obtained. The wet filter cake was washed with fresh 400g methanol. Afterwards, the washed wet filter cake was dried for 5 hours in a Rotavapor^{®} rotary evaporator with a wall temperature of 130°C. The thus obtained product had the following composition:
99,987% 4,4'-DCDPS
120 ppm methanol
90 ppm DCDPS-isomers
< 20 ppm remaining carboxylic acid.

### Example 5 (Working up the solvent used in the DCDPS-production)

The organic phase obtained by phase separation of the mother liquor in example 3 was mixed with 269 g of 50 % sulfuric acid. This mixture was combined with the mother liquor obtained in the filtration in example 2. This combined mother liquor then was separated in two streams. One stream was subjected to distillation and one stream to stripping. After distillation and stripping, respectively, the resulting purified carboxylic acid streams were combined again. The combined mother liquor had the following composition:
0.715 wt% monochlorobenzene, 0.02 wt% dodecane, 0.003 wt% n-heptanoic acid methyl ester, 0.026 wt% valeric acid, 0.315 wt% n-hexanoic acid, 95.02 wt% n-heptanoic acid and 3.5 wt% water.

### Distillation

310 g of the combined mother liquor were fed into a batch distillation column with 10 trays and distilled with a bottom temperature of 160°C, and a top temperature of 135°C at a pressure of 52 mbar (abs) for about 4.5h. The carboxylic acid obtained by this distillation had the following composition:
0.014 wt% monochlorobenzene, 0.002 wt% dodecane, 0.0 wt% n-heptanoic acid methyl ester, 0.005 wt% valeric acid, 0.185 wt% n-hexanoic acid, and 99.52 wt% n-heptanoic acid.

### Stripping

2627 g of the combined mother liquor with a temperature of 88°C were provided in a buffer vessel and continuously fed into a stripping column with a feed rate of 66 ml/min.

The stripping column had 10 trays and the crude carboxylic acid was fed on top into the stripping column and 150 NL per hour nitrogen were fed into the stripping column at the bottom as stripping gas. The pressure in the stripping column was set to 300 mbar.

After stripping, the carboxylic acid was continuously removed from the stripping column and had the following composition:
0.456 wt% monochlorobenzene, 0.018 wt% dodecane, 0.003 wt% n-heptanoic acid methyl ester, 0.025 wt% valeric acid, 0.333 wt% n-hexanoic acid, 95.36 wt% n-heptanoic acid, and 0.42 wt% water.

The purified carboxylic acid (unified from stripping and distillation) was fed back into the oxidation reaction and contained 0.41 wt% monochlorobenzene, 2.2 wt% 4,4'-DCDPS, 0.54% 2,4'-DCDPS, about 600 ppm lactones, 4000 ppm n-hexanoic acid, 240 ppm valerian acid, 100 ppm esters, and 160 ppm dodecane.

## Claims

1. A process for producing 4,4'-dichlorodiphenyl sulfone, comprising:
(I) reacting thionyl chloride, chlorobenzene and aluminum chloride in a molar ratio of thionyl chloride : chlorobenzene : aluminum chloride of 1 : (6 to 9) : (1 to 1.5) at a temperature from 0 to below 20°C, forming an intermediate reaction product and hydrogen chloride;
(II) mixing aqueous hydrochloric acid and the intermediate reaction product at a temperature from 70 to 110°C to obtain an organic phase comprising 4,4'-dichlorodiphenyl sulfoxide and an aqueous phase;
(III) cooling the organic phase comprising the 4,4'-dichlorodiphenyl sulfoxide to a temperature below the saturation point of 4,4'-dichlorodiphenyl sulfoxide to obtain a suspension comprising crystallized 4,4'-dichlorodiphenyl sulfoxide;
(IV) solid-liquid-separation of the suspension to obtain a residual moisture comprising solid 4,4'-dichlorodiphenyl sulfoxide comprising crystallized 4,4'-dichlorodiphenyl sulfoxide and solvent, and mother liquor;
(V) washing the residual moisture comprising solid 4,4'-dichlorodiphenyl sulfoxide with a carboxylic acid to obtain carboxylic acid-wet 4,4'-dichlorodiphenyl sulfoxide;
(VI) reacting the carboxylic acid-wet 4,4'-dichlorodiphenyl sulfoxide and an oxidizing agent in a carboxylic acid as solvent to obtain a reaction mixture comprising 4,4'-dichlorodiphenyl sulfone and carboxylic acid;
(VII) separating the reaction mixture comprising 4,4'-dichlorodiphenyl sulfone and carboxylic acid into a residual moisture comprising 4,4'-dichlorodiphenyl sulfone as crude product and a liquid phase comprising carboxylic acid.
(VIII) optionally working up the residual moisture comprising 4,4'-dichlorodiphenyl sulfone.

2. The process according to claim 1, wherein separating the reaction mixture in (VII) comprises:
(VII.a) mixing the reaction mixture with water in a gastight closed vessel to obtain a liquid mixture, wherein preferably the amount of water mixed to the reaction mixture in (VII.a) is such that the amount of water in the liquid mixture is from 10 to 60 wt% based on the total amount of the liquid mixture;
(VII.b) cooling the liquid mixture obtained in (VII.a) to a temperature below the saturation point of 4,4'-dichlorodiphenyl sulfone by
(i) reducing the pressure in the gastight closed vessel to a pressure at which the water starts to evaporate,
(ii) condensing the evaporated water by cooling
(iii) mixing the condensed water into the liquid mixture in the gastight closed vessel,
to obtain a suspension comprising crystallized 4,4'-dichlorodiphenyl sulfone;
(Vll.c) carrying out a solid-liquid-separation of the suspension to obtain the residual moisture comprising 4,4'-dichlorodiphenyl sulfone and the liquid phase comprising the carboxylic acid.

3. The process according to claim 2, wherein the pressure is reduced in (i) until the suspension has cooled down to a temperature from 10 to 30°C

4. The process according to any of claims 1 to 3, wherein in (VIII) the residual moisture comprising 4,4'-dichlorodiphenyl sulfone is washed with an aqueous base and then with water to obtain a first purified 4,4'-dichlorodiphenyl sulfone.

5. The process according to claim 4, wherein the aqueous base after being used for washing is mixed with a strong acid and a phase separation is carried out in which an aqueous phase and an organic phase comprising the carboxylic acid are obtained, wherein the strong acid preferably is sulfuric acid or alkane sulfonic acid.

6. The process according to any of claims 1 to 5, wherein the carboxylic acid is at least one aliphatic C₆ to C₁₀ carboxylic acid, preferably n-hexanoic acid, n-heptanoic acid or a mixture thereof.

7. The process according to any of claims 1 to 6, wherein cooling of the organic phase comprising 4,4'-dichlorodiphenyl sulfoxide in (III) is carried out in a gastight closed vessel by
(III.a) reducing the pressure in the gastight closed vessel;
(III.b) evaporating solvent;
(III.c) condensing the evaporated solvent by cooling;
(III.d) returning the condensed solvent into the gastight closed vessel.

8. The process according to any of claims 1 to 7, wherein the residual moisture comprising 4,4'-dichlorodiphenyl sulfone or the first purified 4,4'-dichlorodiphenyl sulfone, if the residual moisture comprising 4,4'-dichlorodiphenyl sulfone is washed, is further processed by:
(A) dissolving the residual moisture comprising 4,4'-dichlorodiphenyl sulfone or the first purified 4,4'-dichlorodiphenyl sulfone in an organic solvent in which 4,4'-dichlorodiphenyl sulfone has a solubility of 0.5 to 20% at 20°C to obtain a solution;
(B) cooling the solution to a temperature below the saturation point of 4,4'-dichlorodiphenyl sulfone to obtain a suspension comprising crystallized 4,4'-dichlorodiphenyl sulfone;
(C) carrying out a solid-liquid separation to obtain residual moisture comprising 4,4'-dichlorodiphenyl sulfone and a mother liquor;
(D) washing the residual moisture comprising 4,4'-dichlorodiphenyl sulfone with an organic solvent in which 4,4'-dichlorodiphenyl sulfone has a solubility of 0.5 to 20% at 20°C;
(E) optionally repeating steps (B) to (D);
(F) drying the 4,4'-dichlorodiphenyl sulfone;
(G) optionally working up and preferably recycling into the dissolving (A) at least a part of the mother liquor and optionally the organic solvent in which 4,4'-dichlorodiphenyl sulfone has a solubility of 0.5 to 20% at 20°C used for washing by distillation.

9. The process according to any of claims 1 to 8, wherein the liquid phase comprising carboxylic acid obtained in (VII) is purified by
- distilling a part of the liquid phase comprising carboxylic acid;
- stripping low boilers from at least a part of the liquid phase comprising carboxylic acid; and
- recycling the purified carboxylic acid into the reaction (VI)

10. The process according to any of claims 1 to 9, wherein the residual moisture comprising solid 4,4'-dichlorodiphenyl sulfoxide obtained in (IV) is washed with solvent, wherein preferably at least a part of the solvent is purified after being used for washing the residual moisture comprising solid 4,4'-dichlorodiphenyl sulfoxide and then recycled.

11. The process according to any of claims 1 to 10, wherein the mother liquor obtained in (IV) is concentrated and at least a part of the concentrated mother liquor is recycled into the cooling of the organic phase comprising 4,4'-dichlorodiphenyl sulfoxide (III).

12. The process according to any of claims 1 to 11, wherein the amount of carboxylic acid in the reaction (VI) is such that the weight ratio of 4,4'-dichlorodiphenyl sulfoxide to carboxylic acid is at least 1 : 2.

13. The process according to any of claims 1 to 12, wherein the oxidizing agent is a peroxide.

14. The process according to any of claims 1 to 13, wherein a liquid mixture comprising chlorobenzene and carboxylic acid is withdrawn from the washing (V) and the liquid mixture comprising chlorobenzene and carboxylic acid is separated into a first stream comprising substantially chlorobenzene and a liquid phase comprising substantially carboxylic acid, wherein preferably the liquid phase is recycled into the washing (V) and the first stream is recycled into the reaction (I).

15. The process according to any of claims 1 to 14, wherein the organic phase comprising 4,4'-dichlorodiphenyl sulfoxide obtained in (II) is separated off and washed with water at a temperature from 70 to 110°C before cooling in (III).

## Patentansprüche

1. Verfahren zur Herstellung von 4,4'-Dichlordiphenylsulfon, das Folgendes umfasst:
(I) Umsetzen von Thionylchlorid, Chlorbenzol und Aluminiumchlorid in einem Molverhältnis von Thionylchlorid : Chlorbenzol : Aluminiumchlorid von 1 : (6 bis 9) : (1 bis 1,5) bei einer Temperatur von 0 bis unter 20 °C unter Bildung von einem Reaktionszwischenprodukt und Chlorwasserstoff;
(II) Mischen von wässriger Salzsäure und dem Reaktionszwischenprodukt bei einer Temperatur von 70 bis 110 °C zum Erhalt einer organischen Phase, die 4,4'-Dichlordiphenylsulfoxid umfasst, und einer wässrigen Phase;
(III) Abkühlen der organischen Phase, die das 4,4'-Dichlordiphenylsulfoxid umfasst, auf eine Temperatur unterhalb des Sättigungspunkts von 4,4'-Dichlordiphenylsulfoxid zum Erhalt einer Suspension, die kristallisiertes 4,4'-Dichlordiphenylsulfoxid umfasst;
(IV) Fest-Flüssig-Trennung der Suspension zum Erhalt von Restfeuchtigkeit umfassendem festem 4,4'-Dichlordiphenylsulfoxid, das kristallisiertes 4,4'-Dichlordiphenylsulfoxid und Lösungsmittel umfasst, und Mutterlauge;
(V) Waschen des Restfeuchtigkeit umfassenden festen 4,4'-Dichlordiphenylsulfoxids mit einer Carbonsäure zum Erhalt von carbonsäurefeuchtem 4,4'-Dichlordiphenylsulfoxid;
(VI) Umsetzen des carbonsäurefeuchten 4,4'-Dichlordiphenylsulfoxids und eines Oxidationsmittels in einer Carbonsäure als Lösungsmittel zum Erhalt einer Reaktionsmischung, die 4,4'-Dichlordiphenylsulfon und Carbonsäure umfasst;
(VII) Trennen der Reaktionsmischung, die 4,4'-Dichlordiphenylsulfon und Carbonsäure umfasst, in ein Restfeuchtigkeit umfassendes 4,4'-Dichlordiphenylsulfon als Rohprodukt und eine flüssige Phase, die Carbonsäure umfasst;
(VIII) gegebenenfalls Aufarbeiten des Restfeuchtigkeit umfassenden 4,4'-Dichlordiphenylsulfons.

2. Verfahren nach Anspruch 1, wobei das Trennen der Reaktionsmischung in (VII) Folgendes umfasst:
(VII.a) Mischen der Reaktionsmischung mit Wasser in einem gasdichten verschlossenen Gefäß zum Erhalt einer flüssigen Mischung, wobei vorzugsweise die der Reaktionsmischung in (VII.a) zugemischte Wassermenge so beschaffen ist, dass die Wassermenge in der flüssigen Mischung 10 bis 60 Gew.-%, bezogen auf die Gesamtmenge der flüssigen Mischung, beträgt;
(VII.b) Abkühlen der in (VII.a) erhaltenen flüssigen Mischung auf eine Temperatur unterhalb des Sättigungspunkts von 4,4'-Dichlordiphenylsulfon durch
(i) Verringern des Drucks in dem gasdichten geschlossenen Gefäß auf einen Druck, bei dem das Wasser zu verdampfen beginnt,
(ii) Kondensieren des verdampften Wassers durch Abkühlen,
(iii) Einmischen des kondensierten Wassers in die flüssige Mischung in dem gasdichten verschlossenen Gefäß
zum Erhalt einer Suspension, die kristallisiertes 4,4'-Dichlordiphenylsulfon umfasst;
(VII.c) Durchführen einer Fest-Flüssig-Trennung der Suspension zum Erhalt des Restfeuchtigkeit umfassenden 4,4'-Dichlordiphenylsulfons und der flüssigen Phase, die die Carbonsäure umfasst.

3. Verfahren nach Anspruch 2, wobei der Druck in (i) verringert wird, bis die Suspension auf eine Temperatur von 10 bis 30 °C abgekühlt ist.

4. Verfahren nach einem der Ansprüche 1 bis 3, wobei in (VIII) das Restfeuchtigkeit umfassende 4,4'-Dichlordiphenylsulfon mit einer wässrigen Base und dann mit Wasser gewaschen wird, wobei man ein erstes gereinigtes 4,4'-Dichlordiphenylsulfon erhält.

5. Verfahren nach Anspruch 4, wobei die wässrige Base nach der Verwendung zum Waschen mit einer starken Säure gemischt wird und eine Phasentrennung durchgeführt wird, bei der man eine wässrige Phase und eine organische Phase, die die Carbonsäure umfasst, erhält, wobei es sich bei der starken Säure vorzugsweise um Schwefelsäure oder eine Alkansulfonsäure handelt.

6. Verfahren nach einem der Ansprüche 1 bis 5, wobei es sich bei der Carbonsäure um mindestens eine aliphatische C₆- bis C₁₀-Carbonsäure, vorzugsweise n-Hexansäure, n-Heptansäure oder eine Mischung davon, handelt.

7. Verfahren nach einem der Ansprüche 1 bis 6, wobei das Abkühlen der organischen Phase, die 4,4'-Dichlordiphenylsulfoxid umfasst, in (III) in einem gasdichten geschlossenen Gefäß durch
(III.a) Verringern des Drucks in dem gasdichten geschlossenen Gefäß;
(III.b) Verdampfen von Lösungsmittel;
(III.c) Kondensieren des verdampften Lösungsmittels durch Abkühlen;
(III.d) Zurückführen des kondensierten Lösungsmittels in das gasdichte geschlossene Gefäß durchgeführt wird.

8. Verfahren nach einem der Ansprüche 1 bis 7, wobei das Restfeuchtigkeit umfassende 4,4'-Dichlordiphenylsulfon oder das erste gereinigte 4,4'-Dichlordiphenylsulfon, falls das Restfeuchtigkeit umfassende 4,4'-Dichlordiphenylsulfon gewaschen wird, durch
(A) Lösen des Restfeuchtigkeit umfassenden 4,4'-Dichlordiphenylsulfons bzw. des ersten gereinigten 4,4'-Dichlordiphenylsulfons in einem organischen Lösungsmittel, in dem 4,4'-Dichlordiphenylsulfon eine Löslichkeit von 0,5 bis 20 % bei 20 °C aufweist, zum Erhalt einer Lösung;
(B) Abkühlen der Lösung auf eine Temperatur unterhalb des Sättigungspunkts von 4,4'-Dichlordiphenylsulfon zum Erhalt einer Suspension, die kristallisiertes 4,4'-Dichlordiphenylsulfon umfasst;
(C) Durchführen einer Fest-Flüssig-Trennung zum Erhalt von Restfeuchtigkeit umfassendem 4,4'-Dichlordiphenylsulfon und einer Mutterlauge;
(D) Waschen des Restfeuchtigkeit umfassenden 4,4'-Dichlordiphenylsulfons mit einem organischen Lösungsmittel, in dem 4,4'-Dichlordiphenylsulfon eine Löslichkeit von 0,5 bis 20 % bei 20 °C aufweist;
(E) gegebenenfalls Wiederholen der Schritte (B) bis (D);
(F) Trocknen des 4,4'-Dichlordiphenylsulfons;
(G) gegebenenfalls Aufarbeiten und vorzugsweise Rezyklieren mindestens eines Teils der Mutterlauge und gegebenenfalls des zum Waschen verwendeten organischen Lösungsmittels, in dem 4,4'-Dichlordiphenylsulfon eine Löslichkeit von 0,5 bis 20 % bei 20 °C aufweist, durch Destillation in das Lösen (A)
weiterverarbeitet wird.

9. Verfahren nach einem der Ansprüche 1 bis 8, wobei die in (VII) erhaltene flüssige Phase, die Carbonsäure umfasst, durch
- Destillieren eines Teils der flüssigen Phase, die Carbonsäure umfasst;
- Abstrippen von Niedrigsiedern von mindestens einem Teil der flüssigen Phase, die Carbonsäure umfasst; und
- Rezyklieren der gereinigten Carbonsäure in die Umsetzung (VI)
gereinigt wird.

10. Verfahren nach einem der Ansprüche 1 bis 9, wobei das in (IV) erhaltene Restfeuchtigkeit umfassende feste 4,4'-Dichlordiphenylsulfon mit einem Lösungsmittel gewaschen wird, wobei vorzugsweise mindestens ein Teil des Lösungsmittels nach der Verwendung zum Waschen des Restfeuchtigkeit umfassenden festen 4,4'-Dichlordiphenylsulfons gereinigt und dann rezykliert wird.

11. Verfahren nach einem der Ansprüche 1 bis 10, wobei die in (IV) erhaltene Mutterlauge aufkonzentriert wird und mindestens ein Teil der aufkonzentrierten Mutterlauge zum Abkühlen der organischen Phase, die 4,4'-Dichlordiphenylsulfon umfasst, (III) rezykliert wird.

12. Verfahren nach einem der Ansprüche 1 bis 11, wobei die Carbonsäuremenge bei der Umsetzung (VI) so beschaffen ist, dass das Gewichtsverhältnis von 4,4'-Dichlordiphenylsulfon zu Carbonsäure mindestens 1 : 2 beträgt.

13. Verfahren nach einem der Ansprüche 1 bis 12, wobei es sich bei dem Oxidationsmittel um ein Peroxid handelt.

14. Verfahren nach einem der Ansprüche 1 bis 13, wobei eine flüssige Mischung, die Chlorbenzol und Carbonsäure umfasst, aus dem Waschen (V) abgezogen wird und die flüssige Mischung, die Chlorbenzol und Carbonsäure umfasst, in einen ersten Strom, der weitgehend Chlorbenzol umfasst, und eine flüssige Phase, die weitgehend Carbonsäure umfasst, getrennt wird, wobei vorzugsweise die flüssige Phase zum Waschen (V) rezykliert wird und der erste Strom zur Umsetzung (I) rezykliert wird.

15. Verfahren nach einem der Ansprüche 1 bis 14, wobei die in (II) erhaltene organische Phase, die 4,4'-Dichlordiphenylsulfon umfasst, abgetrennt und vor dem Abkühlen (III) mit Wasser bei einer Temperatur von 70 bis 110 °C gewaschen wird.

## Revendications

1. Procédé pour la production de 4,4'-dichlorodiphénylsulfone, comprenant :
(I) la mise en réaction de chlorure de thionyle, de chlorobenzène et de chlorure d'aluminium en un rapport molaire de chlorure de thionyle : chlorobenzène : chlorure d'aluminium de 1 : (6 à 9) : (1 à 1,5) à une température de 0 à en dessous de 20 °C, formant un produit de réaction intermédiaire et du chlorure d'hydrogène ;
(II) mélange d'acide chlorhydrique aqueux et du produit de réaction intermédiaire à une température de 70 à 110 °C pour obtenir une phase organique comprenant du 4,4'-dichlorodiphénylsulfoxyde et une phase aqueuse ;
(III) refroidissement de la phase organique comprenant le 4,4'-dichlorodiphénylsulfoxyde à une température inférieure au point de saturation du 4,4'-dichlorodiphénylsulfoxyde pour obtenir une suspension comprenant du 4,4'-dichlorodiphénylsulfoxyde cristallisé ;
(IV) séparation solide-liquide de la suspension pour obtenir du 4,4'-dichlorodiphénylsulfoxyde solide comprenant de l'humidité résiduelle comprenant du 4,4'-dichlorodiphénylsulfoxyde cristallisé et du solvant, et une liqueur mère ;
(V) lavage du 4,4'-dichlorodiphényisulfoxyde solide comprenant de l'humidité résiduelle avec un acide carboxylique pour obtenir du 4,4'-dichlorodiphénylsulfoxyde mouillé par un acide carboxylique ;
(VI) mise en réaction du 4,4'-dichlorodiphénylsulfoxyde mouillé par un acide carboxylique est d'un agent oxydant dans un acide carboxylique en tant que solvant pour obtenir un mélange réactionnel comprenant de la 4,4'-dichlorodiphénylsulfone et un acide carboxylique ;
(VII) séparation du mélange réactionnel comprenant de la 4,4'-dichlorodiphénylsulfone et un acide carboxylique en une 4,4'-dichlorodiphénylsulfone comprenant de l'humidité résiduelle en tant que produit brut et une phase liquide comprenant un acide carboxylique,
(VIII) éventuellement, traitement de la 4,4'-dichlorodiphénylsulfone comprenant de l'humidité résiduelle.

2. Procédé selon la revendication 1, la séparation du mélange réactionnel en (VII) comprenant :
(VII.a) le mélange du mélange réactionnel avec de l'eau dans une cuve fermée étanche aux gaz pour obtenir un mélange liquide, préférablement la quantité d'eau mélangée au mélange réactionnel en (VII.a) étant telle que la quantité d'eau dans le mélange liquide soit de 10 à 60 % en poids sur la base de la quantité totale du mélange liquide ;
(VII.b) refroidissement du mélange liquide obtenu en (VII.a) à une température inférieure au point de saturation de la 4,4'-dichlorodiphénylsulfone par
(i) réduction de la pression dans la cuve fermée étanche aux gaz jusqu'à une pression à laquelle l'eau commence à s'évaporer,
(ii) condensation de l'eau évaporée par refroidissement
(iii) mélange de l'eau condensée dans le mélange liquide dans la cuve fermée étanche aux gaz,
pour obtenir une suspension comprenant de la 4,4'-dichlorodiphénylsulfone cristallisée ;
(VII.c) réalisation d'une séparation solide-liquide de la suspension pour obtenir la 4,4'-dichlorodiphénylsulfone comprenant de l'humidité résiduelle et la phase liquide comprenant l'acide carboxylique.

3. Procédé selon la revendication 2, la pression étant réduite en (i) jusqu'à ce que la suspension aie refroidie à une température de 10 à 30 °C.

4. Procédé selon l'une quelconque des revendications 1 à 3, dans lequel en (VIII) la 4,4'-dichlorodiphénylsulfone comprenant de l'humidité résiduelle est lavée avec une base aqueuse et ensuite avec de l'eau pour obtenir une première 4,4'-dichlorodiphénylsulfone purifiée.

5. Procédé selon la revendication 4, la base aqueuse, après avoir été utilisée pour le lavage, étant mélangée avec un acide fort et une séparation de phase étant réalisée dans laquelle une phase aqueuse et une phase organique comprenant l'acide carboxylique sont obtenues, l'acide fort étant préférablement l'acide sulfurique ou un acide alcanesulfonique.

6. Procédé selon l'une quelconque des revendications 1 à 5, l'acide carboxylique étant au moins un acide carboxylique aliphatiquement C₆ à C₁₀, préférablement l'acide n-hexanoïque, l'acide n-heptanoïque ou un mélange correspondant.

7. Procédé selon l'une quelconque des revendications 1 à 6, le refroidissement de la phase organique comprenant du 4,4'-dichlorodiphénylsulfoxyde en (III) étant réalisé dans une cuve fermée étanche aux gaz
(III.a) en réduisant la pression dans la cuve fermée étanche aux gaz ;
(III.b) en évaporant du solvant ;
(III.c) en condensant le solvant évaporé par refroidissement ;
(III.d) en retournant le solvant condensé dans la cuve fermée étanche aux gaz.

8. Procédé selon l'une quelconque des revendications 1 à 7, la 4,4'-dichlorodiphénylsulfone comprenant de l'humidité résiduelle ou la première 4,4'-dichlorodiphénylsulfone purifiée, si la 4,4'-dichlorodiphénylsulfone comprenant de l'humidité résiduelle est lavée, étant en outre traitée en :
(A) dissolvant la 4,4'-dichlorodiphénylsulfone comprenant de l'humidité résiduelle ou la première 4,4'-dichlorodiphénylsulfone purifiée dans un solvant organique dans lequel la 4,4'-dichlorodiphénylsulfone possède une solubilité de 0,5 à 20 % à 20 °C pour obtenir une solution ;
(B) refroidissant la solution à une température inférieure au point de saturation de la 4,4'-dichlorodiphénylsulfone pour obtenir une suspension comprenant de la 4,4'-dichlorodiphénylsulfone cristallisée ;
(C) réalisant une séparation solide-liquide pour obtenir de la 4,4'-dichlorodiphénylsulfone comprenant de l'humidité résiduelle et une liqueur mère ;
(D) lavage de la 4,4'-dichlorodiphénylsulfone comprenant de l'humidité résiduelle avec un solvant organique dans lequel la 4,4'-dichlorodiphénylsulfone possède une solubilité de 0,5 à 20 % à 20 °C ;
(E) éventuellement, répétition des étapes (B) à (D) ;
(F) séchage de la 4,4'-dichlorodiphénylsulfone ;
(G) éventuellement, traitement et préférablement recyclage dans la dissolution (A) d'au moins une partie de la liqueur mère et éventuellement du solvant organique dans lequel la 4,4'-dichlorodiphénylsulfone possède une solubilité de 0,5 à 20 % à 20 °C utilisé pour le lavage, par distillation.

9. Procédé selon l'une quelconque des revendications 1 à 8, la phase liquide comprenant un acide carboxylique obtenue en (VII) étant purifiée par
- distillation d'une partie de la phase liquide comprenant un acide carboxylique ;
- strippage de substances à bas point d'ébullition d'au moins une partie de la phase liquide comprenant un acide carboxylique ; et
- recyclage de l'acide carboxylique purifié dans la réaction (VI).

10. Procédé selon l'une quelconque des revendications 1 à 9, le 4,4'-dichlorodiphénylsulfoxyde solide comprenant de l'humidité résiduelle obtenu en (IV) étant lavé avec du solvant, préférablement au moins une partie du solvant étant purifiée après avoir été utilisée pour le lavage du 4,4'-dichlorodiphénylsulfoxyde solide comprenant de l'humidité résiduelle et ensuite recyclée.

11. Procédé selon l'une quelconque des revendications 1 à 10, la liqueur mère obtenue en (IV) étant concentrée et au moins une partie de la liqueur mère concentrée étant recyclée dans le refroidissement de la phase organique comprenant du 4,4'-dichlorodiphénylsulfoxyde (III).

12. Procédé selon l'une quelconque des revendications 1 à 11, la quantité d'acide carboxylique dans la réaction (VI) étant telle que le rapport en poids de 4,4'-dichlorodiphénylsulfoxyde sur acide carboxylique est d'au moins 1 : 2.

13. Procédé selon l'une quelconque des revendications 1 à 12, l'agent oxydant étant un peroxyde.

14. Procédé selon l'une quelconque des revendications 1 à 13, un mélange liquide comprenant du chlorobenzène et un acide carboxylique étant soutiré du lavage (V) et le mélange liquide comprenant du chlorobenzène et un acide carboxylique étant séparé en un premier flux comprenant sensiblement du chlorobenzène et une phase liquide comprenant sensiblement de l'acide carboxylique, préférablement la phase liquide étant recyclée dans le lavage (V) et le premier flux étant recyclé dans la réaction (I).

15. Procédé selon l'une quelconque des revendications 1 à 14, la phase organique comprenant du 4,4'-dichlorodiphénylsulfoxyde obtenue en (II) étant séparée et lavée avec de l'eau à une température de 70 à 110 °C avant refroidissement en (III).
